# EUROPEAN PATENT SPECIFICATION

(11) **EP 4 067 354 B1**
(45) Date of publication and mention of the grant of the patent: **22.10.2025**
(21) Application number: 20892809.3
(22) Date of filing: 25.11.2020
(51) Int. Cl.: C07D 471/04, C07D 519/00, A61K 31/4439, A61P 35/00, A61P 35/02, A61P 37/00

(54) **NOVEL TRIAZOLOPYRIDINE DERIVATIVE AND PHARMACEUTICAL COMPOSITION COMPRISING SAME**
NEUARTIGE TRIAZOLOPYRIDINDERIVATE UND DIESE ENTHALTENDE PHARMAZEUTISCHE ZUSAMMENSETZUNG
NOUVEAU DÉRIVÉ DE TRIAZOLOPYRIDINE ET COMPOSITION PHARMACEUTIQUE LE COMPRENANT

(30) Priority: 25.11.2019 KR 20190152660
(43) Date of publication of application: 05.10.2022
(73) Proprietor: Daewoong Pharmaceutical Co., Ltd., Hwaseong-si, Gyeonggi-do 18623 (KR)
(72) Inventor: KIM, Ji Duck, Hwaseong-si, Gyeonggi-do 18454 (KR); JUN, Sun Ah, Yongin-si, Gyeonggi-do 17027 (KR); KIM, Nam Youn, Yongin-si, Gyeonggi-do 17004 (KR); KIM, In Woo, Seoul 08284 (KR); HYUN, Hyae Jung, Seongnam-si, Gyeonggi-do 13568 (KR); PARK, Joon Seok, Yongin-si, Gyeonggi-do 17000 (KR)
(74) Representative: Germain Maureau
(86) International application number: PCT/KR2020/016798
(87) International publication number: WO 2021/107590

(56) References cited:
- WO-A1-2010/010184
- WO-A1-2010/010188
- KR-A- 20110 033 223
- KR-A- 20120 087 936
- KR-A- 20130 091 464

## Description

### [Technical Field]

The present invention relates to a novel triazolopyridine derivative having kinase inhibitory activity, and a pharmaceutical composition for use in the prevention or treatment of inflammatory disease, autoimmune disease, proliferative disease, hyperproliferative disease, immunity mediated disease, cancer or tumor, comprising the same or a pharmaceutically acceptable salt thereof.

### [Background Art]

Protein kinase is an enzyme that catalyzes phosphorylation of specific residues of other proteins, and plays an important role in signal-transduction pathways that transduce extracellular signals to the nucleus. Further, it is involved in various diseases in vivo. In the onset or development of inflammatory disease, autoimmune disease, proliferative disease or hyperproliferative disease, and/or immunity mediated disease, there is various evidence that T-cells (or T-lymphocytes) and B-cells (or B-lymphocytes) play an important role.

Janus kinase (hereinafter referred to as "JAK") is a cytoplasmic protein tyrosine kinase that plays pivotal roles in regulating cell function in the lympho-hematopoietic system. Cytokines are known to play an important role in regulating inflammation, immunity and normal cell function, and JAK activates STAT (Signal Transducer and Activators of Transcription) proteins through tyrosine phosphorylation to provide rapid signaling pathways to cytokines. JAK/STAT signaling is known to be associated with allergies, asthma, autoimmune diseases (e.g., transplant rejection, rheumatoid arthritis, amyotrophic lateral sclerosis, multiple sclerosis etc.), solid cancers, blood cancers (e.g., leukemia, lymphoma and so on).

The JAK family is classified into four members: JAK 1, JAK 2, JAK 3, and TYK 2. Members of the JAK family pair with each other to mediate signals from a variety of cytokines. It includes JAK2 and JAK1 associated with hematopoietic growth factor signaling, and a combination of TYK2 and AK2 is important for interferon signaling and contributes to host tolerance. JAK2 can induce anemia, thrombocytopenia, leukopenia, especially when it is involved in the hematopoietic growth factor signaling and causes excessive inhibition.

The expression of JAK1, JAK2, and TYK2 was found to be widely distributed, whereas the expression of JAK3 was restricted to lymphocytes and is associated with signaling for the common gamma chains, members of IL-2, IL-4, IL-7, IL-9, IL-15 and IL-21 receptors, particularly the common gamma chain of the IL-2 family. As soon as the cytokine is bound, the receptor carries adjacent JAK3 nearby, which induces autophosphorylation of the β-chain C-terminus. As a result, it causes activation of the STAT protein, which is an important step in retransmitting the signal to the nucleus. JAK3 controls the signal pathways of various cytokines through this process. This makes JAK3 as an attractive target for immunosuppression.

B cells play an important role in the development of autoimmune and/or inflammatory diseases. Protein-based therapeutic agents that reduce B cells, for example Rituxan, are effective in autoantibody-induced inflammatory diseases such as rheumatoid arthritis. Thus, protein kinase inhibitors that play a role in B cell activation are useful therapeutic agents for the treatment of B cell-mediated diseases, for example, for the production of autoantibodies.

Signal transduction through B cell receptor (BCR) regulates various B cell responses, including proliferation and differentiation into mature antibody-producing cells. BCR is an important regulatory element of B cell activity, and abnormal signal transduction can cause the formation of pathogenic autoantibodies leading to a plurality of autoimmune and/or inflammatory diseases and the proliferation of deregulated B cell.

Bruton's tyrosine kinase (hereinafter, referred to as "BTK") is an important regulator of the development, activation, signaling and survival of B-cells. BTK is involved in signal transduction pathways initiated by binding various extracellular ligands to their cell surface receptors. Following ligation of the B cell antigen receptor (BCR), the activity of BTK by the coincident action of the protein tyrosine kinases Lyn and Syk is required for the induction of the phospholipase C-γ2-mediated calcium mobilization. Therefore, inhibition of BTK can be a useful therapeutic approach in blocking the onset process of B-cell mediated diseases.

As mentioned above, Janus kinase and TEC-based kinases plays an important role in the activation of T-cells and/or B-cells involved in the development of inflammatory diseases, autoimmune diseases, proliferative diseases or hyperproliferative diseases, and immunity mediated diseases. Therefore, the development of substances that effectively inhibit these diseases can be useful as a related therapeutic agent. Specific examples of the diseases which can be treated and prevented include cancer, transplant rejection, multiple sclerosis, rheumatoid arthritis, psoriatic arthritis, psoriasis, asthma, allergic dermatitis, atopic dermatitis, eczema, type I diabetes, diabetic complication, ulcerative colitis, Crohn's disease, autoimmune thyroid disorder, systemic depilation and Sjogren's syndrome.

JAK3 kinase inhibitor, tofacitinib (CP-690550) (Pfizer Inc.) is currently approved and marketed for the treatment of rheumatoid arthritis. In addition, a BTK kinase inhibitor, ibrutinib (PCI-32765) (Pharmacyclics) is in a clinical stage, but severe side effects such as skin rash and diarrhea have been reported in clinical cases. Thus, there is a need to develop a more stable and effective substance that inhibits JAK and/or BTK (see, Nat Rev Rheumatol. 2009 Jun 5(6) 317-24; Expert Opin Investig Drugs. 2014 Aug 23(8) 1067-77; Drug Discov Today 2014 Aug 19(8) 1200-4; WO2002/096909; WO2010-009342).

KR2013-0091464A discloses a pharmaceutical composition for preventing or treating inflammatory diseases, autoimmune diseases, proliferative diseases or hyperproliferative diseases, immune-mediated diseases, cancer or tumors, comprising a triazolopyridine derivative having the following formula

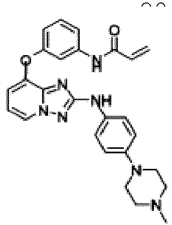

WO2010/010188A1 and WO2010/010184A1 disclose [1,2,4]triazolo[1,5-a]pyridine compounds which may be used for the prevention and treatment of diseases involving cartilage degradation, bone and/or joint degradation; and/or conditions involving inflammation or immune responses, such as Crohn's disease, rheumatoid arthritis, psoriasis, allergic airways disease, juvenile idiopathic arthritis, colitis, inflammatory bowel diseases, endotoxin-driven disease states, diseases involving impairment of cartilage tumover, congenital cartilage malformations, diseases associated with hypersecretion of IL6 and transplantation rejection and proliferative diseases.

KR2012-0087936A discloses compounds that are inhibitors of 3-phosphoinositide-dependent protein kinase-1 (PDK1) for treating cancer.

KR2011-0033223A discloses triazolopyridin compounds that are inhibitors of Janus kinases JAK1, JAK2, JAK3 and TYK2 activity and which may be used for treating cancer, stroke, diabetes, hepatomegaly, cardiovascular disease, multiple sclerosis, Alzheimer's disease, cystic fibrosis, viral disease, autoimmune diseases, atherosclerosis, restenosis, psoriasis, allergic disorders, inflammation, inflammatory diseases, neurological disorders, a neurodegenerative disease, a hormone-related disease, conditions associated with organ transplantation, immunodeficiency disorders, destructive bone disorders, proliferative disorders, infectious diseases, conditions associated with cell death, thrombin-induced platelet aggregation, liver disease, pathologic immune conditions involving T cell activation, CNS disorders or a myeloproliferative disorder.

Therefore, the present inventors have found a novel triazolopyridine derivative having an excellent inhibitory activity as a kinase inhibitor, thereby completing the present invention.

### [Disclosure]

### [Technical Problem]

It is an object of the present invention to provide novel triazolopyridine derivative having kinase inhibitory activity, and a pharmaceutical composition comprising the same.

### [Technical Solution]

In order to achieve the above objects, according to the present invention, there is provided a compound represented by the following Chemical Formula 1, or a pharmaceutically acceptable salt thereof: 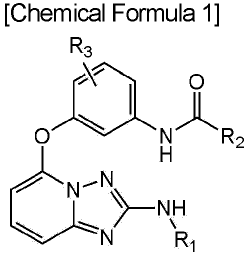 in Chemical Formula 1,
R₁ is C₆₋₁₀aryl, or C₃₋₁₀heteroaryl containing 1 to 3 heteroatoms each independently selected from the group consisting of **N,** O and S,
wherein R₁ is unsubstituted, or substituted with: C₁₋₄alkyl, C₁₋₄alkyl substituted with N(C₁₋₄alkyl)₂, C₁₋₄alkyl substituted with morpholino, C₁₋₄haloalkyl, halogen, C₁₋₄alkoxy, C₁₋₄ alkoxy substituted with N(C₁₋₄ alkyl)₂, N(C₁₋₄ alkyl)₂, morpholino, morpholinocarbonyl, phenoxy, piperidinyl, -SO₂-piperidinyl, piperazinyl, piperazinyl substituted with C₁₋₄ alkyl, benzyl, benzyl substituted with C₁₋₄alkoxy, pyrazolyl, pyrazolyl substituted with one or two C₁₋₄alkyl, tetrazolyl, or tetrazolyl substituted with C₁₋₄ alkyl,
R₂ is C₁₋₅ alkyl, C₂₋₅ alkenyl, or C₂₋₅ alkynyl,
wherein R₂ is unsubstituted, or substituted with one or two substituents selected from the group consisting of halogen, cyano, C₃₋₆ cycloalkyl and C₁₋₅ alkyl substituted with cyano, and
R₃ is hydrogen, halogen, C₁₋₄ alkyl, C₁₋₄ haloalkyl, or C₁₋₄ alkoxy.

Preferably, the Chemical Formula 1 may be represented by the following Chemical Formula 1-1: 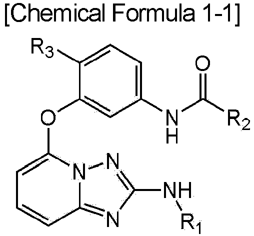 in Chemical Formula 1-1,
R₁ to R₃ are as defined in Chemical Formula 1.

Preferably, R₁ may be benzothiazolyl, imidazo[4,5-b]pyridinyl, isoxazolo[5,4-b]pyridinyl, naphthyl, phenyl, pyrazolyl, pyridinyl, or quinolinyl.

Preferably, R₁ is phenyl, wherein R₁ may be substituted with: C₁₋₄alkyl substituted with N(C₁₋₄ alkyl)₂, C₁₋₄ alkoxy substituted with N(C₁₋₄ alkyl)₂, N(C₁₋₄ alkyl)₂, morpholino, morpholinocarbonyl, phenoxy, -SO₂-piperidinyl, piperazinyl substituted with C₁₋₄ alkyl, pyrazolyl substituted with two C₁₋₄alkyl, or tetrazolyl substituted with C₁₋₄ alkyl.

Preferably, R₁ is pyrazolyl, wherein R₁ may be substituted with: C₁₋₄alkyl, C₁₋₄alkyl substituted with morpholino, C₁₋₄haloalkyl, or benzyl substituted with C₁₋₄alkoxy.

Preferably, R₁ is unsubstituted, or may be benzothiazolyl substituted with halogen; imidazo[4,5-b]pyridinyl substituted with C₁₋₄ alkyl; isoxazolo[5,4-b]pyridinyl substituted with C₁₋₄ alkyl; unsubstituted naphthyl; pyridinyl substituted with morpholino; or unsubstituted quinolinyl, and
more preferably, R₁ is unsubstituted, or may be benzothiazolyl substituted with chloro; imidazo[4,5-b]pyridinyl substituted with methyl; isoxazolo[5,4-b]pyridinyl substituted with methyl; unsubstituted naphthyl; pyridinyl substituted with morpholino; or unsubstituted quinolinyl.

Preferably, R₂ may be any one selected from the group consisting of the following:

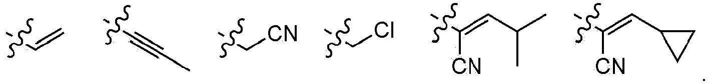

More preferably, R₂ may be any one selected from the group consisting of the following:

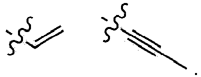

Preferably, R₃ may be hydrogen, fluoro, chloro, methyl, trifluoromethyl, or methoxy.

More preferably, R₃ may be hydrogen or fluoro.

Typical examples of the compounds represented by Chemical Formula 1 are as follows:
1) N-(3-(2-(4-(4-methylpiperazin-1-yl)phenylamino)-[1,2,4]triazolo[1,5-a]pyridin-5-yloxy)phenyl)acrylamide,
2) N-(3-(2-(1-methyl-1H-pyrazol-4-ylamino)-[1,2,4]triazolo[1,5-a]pyridin-5-yloxy)phenyl)acrylamide,
3) N-(3-(2-(benzo[d]thiazol-6-ylamino)-[1,2,4]triazolo[1,5-a]pyridin-5-yloxy)phenyl)acrylamide,
4) N-(3-(2-(4-morpholinophenylamino)-[1,2,4]triazolo[1,5-a]pyridin-5-yloxy)phenyl)acrylamide,
5) N-(3-(2-(3-phenoxyphenylamino)-[1,2,4]triazolo[1,5-a]pyridin-5-yloxy)phenyl)acrylamide,
6) N-(3-(2-(4-(dimethylamino)phenylamino)-[1,2,4]triazolo[1,5-a]pyridin-5-yloxy)phenyl)acrylamide,
7) N-(3-(2-(3-(5-methyl-1H-tetrazol-1-yl)phenylamino)-[1,2,4]triazolo[1,5-a]pyridin-5-yloxy)phenyl)acrylamide,
8) N-(3-(2-(1-(difluoromethyl)-1H-pyrazol-4-ylamino)-[1,2,4]triazolo[1,5-a]pyridin-5-yloxy)phenyl)acrylamide,
9) N-(3-(2-(3-(3,5-dimethyl-1H-pyrazol-1-yl)phenylamino)-[1,2,4]triazolo[1,5-a]pyridin-5-yloxy)phenyl)acrylamide,
10) N-(3-(2-(4-(3,5-dimethyl-1H-pyrazol-1-yl)phenylamino)-[1,2,4]triazolo[1,5-a]pyridin-5-yloxy)phenyl)acrylamide,
11) N-(3-(2-(2-chlorobenzo[d]thiazol-6-ylamino)-[1,2,4]triazolo[1,5-a]pyridin-5-yloxy)phenyl)acrylamide,
12) N-(3-(2-(benzo[d]thiazol-5-ylamino)-[1,2,4]triazolo[1,5-a]pyridin-5-yloxy)phenyl)acrylamide,
13) N-(3-(2-(4-phenoxyphenylamino)-[1,2,4]triazolo[1,5-a]pyridin-5-yloxy)phenyl)acrylamide,
14) N-(3-(2-(1-(3-methoxybenzyl)-1H-pyrazol-4-ylamino)-[1,2,4]triazolo[1,5-a]pyridin-5-yloxy)phenyl)acrylamide,
15) N-(3-(2-(4-(2-(diethylamino)ethoxy)phenylamino)-[1,2,4]triazolo[1,5-a]pyridin-5-yloxy)phenyl)acrylamide,
16) N-(3-(2-(4-((dimethylamino)methyl)phenylamino)-[1,2,4]triazolo[1,5-a]pyridin-5-yloxy)phenyl)acrylamide,
17) N-(3-(2-(4-(morpholine-4-carbonyl)phenylamino)-[1,2,4]triazolo[1,5-a]pyridin-5-yloxy)phenyl)acrylamide,
18) N-(3-(2-(4-(piperidin-1-ylsulfonyl)phenylamino)-[1,2,4]triazolo[1,5-a]pyridin-5-yloxy)phenyl)acrylamide,
19) N-(3-(2-(1-(2-morpholinoethyl)-1H-pyrazol-4-ylamino)-[1,2,4]triazolo[1,5-a]pyridin-5-yloxy)phenyl)acrylamide,
20) N-(3-(2-(4-(4-methylpiperazin-1-yl)phenylamino)-[1,2,4]triazolo[1,5-a]pyridin-5-yloxy)phenyl)but-2-ynamide,
21) N-(3-(2-(6-morpholinopyridin-3-ylamino)-[1,2,4]triazolo[1,5-a]pyridin-5-yloxy)phenyl)acrylamide,
22) N-(3-(2-(6-chlorobenzo[d]thiazol-2-ylamino)-[1,2,4]triazolo[1,5-a]pyridin-5-yloxy)phenyl)acrylamide,
23) N-(3-(2-(naphthalen-1-ylamino)-[1,2,4]triazolo[1,5-a]pyridin-5-yloxy)phenyl)acrylamide,
24) N-(3-(2-(quinolin-3-ylamino)-[1,2,4]triazolo[1,5-a]pyridin-5-yloxy)phenyl)acrylamide,
25) N-(3-(2-(3-methylisoxazolo[5,4-b]pyridin-5-ylamino)-[1,2,4]triazolo[1,5-a]pyridin-5-yloxy)phenyl)acrylamide,
26) N-(3-(2-(1-methyl-1H-imidazo[4,5-b]pyridin-6-ylamino)-[1,2,4]triazolo[1,5-a]pyridin-5-yloxy)phenyl)acrylamide,
27) N-(4-fluoro-3-(2-(4-morpholinophenylamino)-[1,2,4]triazolo[1,5-a]pyridin-5-yloxy)phenyl)acrylamide,
28) N-(3-(2-(benzo[d]thiazol-6-ylamino)-[1,2,4]triazolo[1,5-a]pyridin-5-yloxy)-4-fluorophenyl)acrylamide,
29) N-(4-fluoro-3-(2-(4-(4-methylpiperazin-1-yl)phenylamino)-[1,2,4]triazolo[1,5-a]pyridin-5-yloxy)phenyl)acrylamide,
30) N-(4-fluoro-3-(2-(1-methyl-1H-pyrazol-4-ylamino)-[1,2,4]triazolo[1,5-a]pyridin-5-yloxy)phenyl)acrylamide, and
31) N-(3-(2-(1-(difluoromethyl)-1H-pyrazol-4-ylamino)-[1,2,4]triazolo[1,5-a]pyridin-5-yloxy)-4-fluorophenyl)acrylamide.

In addition, the compounds of the present invention may exist in the form of salts, especially pharmaceutically acceptable salts. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to ae patient and activates effectively and whose side effects do not degrade the beneficial efficacy of the above compound.

Pharmaceutically acceptable salts can be obtained by conventional methods using inorganic or organic acids. For example, the pharmaceutically acceptable salt can be prepared by dissolving the compound represented by Chemical Formula 1 in a water-miscible organic solvent. e.g., acetone, methanol, ethanol or acetonitrile, followed by adding an organic acid or an inorganic acid, and filtering and drying the precipitated crystals. Alternatively, it may be prepared by removing a solvent or an excessive amount of acid from the acid-added reaction mixture under reduced pressure, followed by drying the residue, or by adding a different organic solvent and then filtering the precipitated salt. At this time, the preferred salts may include salts derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid or toluenesulfonic acid.

A pharmaceutically unacceptable salt or solvate of the compound of Chemical Formula 1 may be used as an intermediate when preparing the compound of Chemical Formula 1, or the pharmaceutically acceptable salt or the solvate thereof.

The compound of Chemical Formula 1 according to the present invention includes not only pharmaceutically acceptable salts thereof, but all solvates and hydrates that can be prepared therefrom, and includes all possible stereoisomers as well. The solvate, the hydrate and the stereoisomer of the compound of Chemical Formula 1 may be prepared and used from the compound of Chemical Formula 1 using common methods.

In addition, the compound of Chemical Formula 1 according to the present invention may be prepared either in a crystalline form or in a non-crystalline form, and when the compound of Chemical Formula 1 is prepared in a crystalline form, it may be optionally hydrated or solvated. In the present invention, the compound of Chemical Formula 1 may not only include a stoichiometric hydrate, but include a compound containing various amounts of water. The solvate of the compound of Chemical Formula 1 according to the present invention includes both stoichiometric solvates and non-stoichiometric solvates.

Furthermore, as an example, the present invention can produce the compound represented by Chemical Formula 1 through Reaction Scheme 1 below. 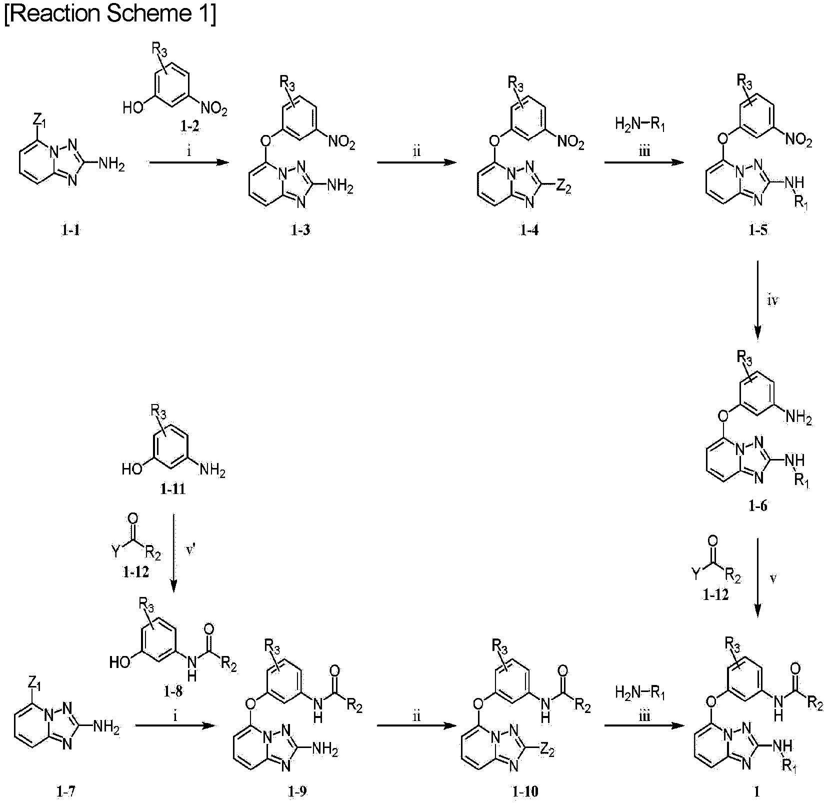 (in Reaction Scheme 1, R₁ to R₃ are as previously defined, and Z₁ and Z₂ are each independently halogen. Y is halogen or OH)

Step i is a step of preparing a compound represented by Chemical Formula 1-3 or Chemical Formula 1-9 by reacting a compound represented by Chemical Formula 1-1 or Chemical Formula 1-7 with a compound represented by Chemical Formula 1-2 or Chemical Formula 1-8. The reaction is preferably carried out in the presence of cesium carbonate at room temperature to high temperature, and the solvent is preferably dimethylformamide.

Step ii is a step of preparing a compound represented by Chemical Formula 1-4 or Chemical Formula 1-10 by halogenating a compound represented by Chemical Formula 1-3 or Chemical Formula 1-9. The reaction is preferably carried out under at room temperature under conditions of potassium iodide, sodium nitrite, and para-toluenesulfonic acid, and the solvent is preferably a mixture of acetonitrile and water.

Step iii is a step of preparing a compound represented by Chemical Formula 1-5 or Chemical Formula 1 by reacting a compound represented by Chemical Formula 1-4 or Chemical Formula 1-10 with R₁-NH₂. The reaction is preferably carried out at 50°C to 150°C under basic conditions in the presence of a palladium catalyst which is tris(dibenzylideneacetone)dipalladium (Pd₂(dba)₃), palladium acetate (Pd(OAc)₂), [1,1'-bis(diphenylphosphine)ferrocene]palladium dichloride ((Pd(Dppf)Cl₂), and the solvent is preferably dioxane, toluene, or dimethylformamide. The alkali used in the reaction is preferably an inorganic alkali such as cesium carbonate, calcium carbonate, sodium carbonate or potassium phosphate.

Step iv is a step of preparing a compound represented by Chemical Formula 1-6 by subjecting a compound represented by Chemical Formula 1-5 to reduction reaction. The reaction is preferably carried out by hydrogenation in a mixed solution of methanol and chloroform in the presence of 10% palladium/carbon. Further, the compound can be prepared by using a metal (iron) or a metal salt (stannous chloride) in hydrochloric acid or ethanol.

Steps v and v' are a step of preparing a compound represented by Chemical Formula 1 or 1-8 by reacting a compound represented by Chemical Formula 1-6 or 1-11 with a compound represented by Chemical Formula 1-12. In Steps v and v', preferably, Y may be Cl or OH. When Y is Cl, the reaction is preferably carried out at -20°C to 0°C under conditions of triethylamine or sodium hydrogen carbonate, and the solvent is preferably a mixture of dichloromethane or tetrahydrofuran and water. When Y is OH, the reaction is preferably carried out at room temperature for 24 hours under conditions of N,N-diisopropylethylamine, 1-[bis(dimethylamino)methylene]-1H-1,2,3-triazolo[4,5-b]pyridinium 3-oxide hexafluorophosphate, and the solvent is preferably tetrahydrofuran, dimethylformamide or dimethyl sulfoxide.

Meanwhile, in step v, when R₂ has a cyano substituent, the reaction may be carried out by the step of preparing the compound represented by Formula 1 by reacting the compound represented by Chemical Formula 1-6 with the compound represented by Chemical Formula 1-12 wherein Y is OH, and then reacting an aldehyde under piperidine conditions. Preferably, the reaction may be carried out at 25°C under conditions of (1-[bis(dimethylamino)methylene]-1H-1,2,3-triazolo[4,5-b]pyridinium 3-oxide hexafluorophosphate), and N,N-diisopropylethylamine, and then reacted with an aldehyde under piperidine conditions. The solvent is preferably dimethylformamide or methanol.

Further, according to the present invention, there is provided a pharmaceutical composition for use in preventing or treating diseases which are associated with kinase inhibitory actions selected from inflammatory disease, autoimmune disease, proliferative disease, hyperproliferative disease, immunity mediated disease, cancer or tumor, comprising the compound represented by Chemical Formula 1, or a pharmaceutically acceptable salt, hydrate, solvate or isomer thereof as an active ingredient.

In this case, the diseases which are associated with kinase inhibitory actions includes inflammatory diseases, autoimmune diseases, proliferative diseases, hyperproliferative diseases, and immunity mediated diseases.

As used herein, the term "prevention" refers to any act to delay or inhibit occurrence, spread or recurrence of the above-mentioned diseases by administration of the composition of the present invention, and "treatment" refers to any act to improve or change the symptoms of the above diseases for the better by administration of the composition of the present invention.

The pharmaceutical composition according to the present invention can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate. Diluents include, for example, lactose, dextrose, sucrose, mannitol, sorbitol, cellulose and/or glycine, but are not limited thereto. Further, the compounds of the present invention can be dissolved in oils, propylene glycol or other solvents commonly used in the preparation of injection solutions. Furthermore, the compounds of the present invention can be formulated in ointments or creams for topical application.

Pharmaceutical dosage forms of the compounds of the present invention may include using the compounds in the form of pharmaceutically acceptable salts or solvates thereof, and using the compounds alone or as a combination and/or a suitable mixture together with other pharmaceutically active compounds.

The compounds of the present invention can be formulated into injection solutions by dissolving, suspending or emulsifying the compounds in a water-soluble solvent such as normal saline, 5% dextrose or a non-aqueous solvent such as synthetic fatty acid glyceride, higher fatty acid ester or propylene glycol. Formulations of the present invention may include conventional additives such as solubilizers, isotonic agents, suspending agents, emulsifying agents, stabilizers and preservatives.

A preferred dose of the compound of the present invention may be varied according to the condition and weight of a patient, the severity of a disease, the type of a drug, and the route and duration of administration, but it may be suitably selected by those skilled in the art. In order to achieve the desirable effects, however, the compound of the present invention may be administrated daily at a dose of 0.0001 to 100 mg/kg (body weight), and preferably 0.001 to 100 mg/kg (body weight). The administration may be performed once a day or in divided doses each day through an oral or parenteral route. Depending on the method of administration, the composition may contain the compound of the present invention in an amount of 0.001 to 99% by weight, preferably 0.01 to 60% by weight.

The pharmaceutical composition according to the present invention may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

The compound represented by Chemical Formula 1 according to the present invention or a pharmaceutically acceptable salt, hydrate, solvate or isomer thereof can be usefully used for the prevention or treatment of diseases which are associated with kinase inhibitory actions

### [Mode For Invention]

Below, the present invention will be described in more detail to assist in the understanding of the invention, in the following examples provided for illustrative purposes.

### Example 1: Preparation of N-(3-(2-(4-(4-methylpiperazin-1-yl)phenylamino)-[1,2,4]triazolo[1,5-a]pyridin-5-yloxy)phenyl)acrylamide

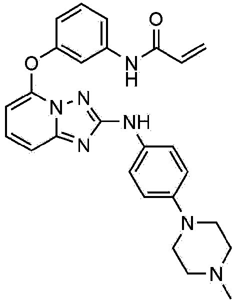

### Step 1) Preparation of 5-(3-nitrophenoxy)-[1,2,4]triazolo[1,5-a]pyridin-2-amine

After 5-bromo-[1,2,4]triazolo[1,5-a]pyridin-2-amine (800.0 mg, 3.8 mmol) was dissolved in N,N-dimethylformamide (5.0 mL), cesium carbonate (2447.0 mg, 7.5 mmol) and 3-nitrophenol (784.0 mg, 5.6 mmol) were added thereto. The reaction mixture was heated at 100°C for 60 hours. Distilled water was added thereto and the solution was stirred for 10 minutes. The organic layer was separated, treated with magnesium sulfate, filtered, and concentrated under reduced pressure. Ethyl acetate (2.0 mL) was added to the residue, and then stirred for 10 minutes. The solid was filtered to obtain 5-(3-nitrophenoxy)-[1,2,4]triazolo[1,5-a]pyridin-2-amine (766.0 mg, yield: 75.2%).

¹H NMR (500MHz, CD₃OD) δ 8.15 (d, 1H), 8.03 (s, 1H), 7.70 (t, 1H), 7.57-7.52 (m, 2H), 7.23 (d, 1H), 6.50 (d, 1H)

### Step 2) Preparation of 2-iodo-5-(3-nitrophenoxy)-[1,2,4]triazolo[1,5-a]pyridine

After 5-(3-nitrophenoxy)-[1,2,4]triazolo[1,5-a]pyridin-2-amine (500.0 mg, 1.8 mmol) and toluenesulfonic acid (1052.0 mg, 5.5 mmol) were dissolved in acetonitrile (20.0 mL), a solution of potassium iodide (765.0 mg, 4.6 mmol) and sodium nitrite (254.0 mg, 3.7 mmol) in distilled water (1.0 mL) was added dropwise thereto. The reaction mixture was stirred at room temperature for 20 hours, and then distilled water was added thereto. The organic layer was separated, treated with magnesium sulfate, filtered, and then concentrated under reduced pressure. The residue was separated by column chromatography to obtain 2-iodo-5-(3-nitrophenoxy)-[1,2,4]triazolo[1,5-a]pyridine (372.0 mg, yield: 52.8%).

¹H NMR (500MHz, CD₃OD) δ 8.21 (dd, 1H), 8.15-8.14 (m, 1H), 7.75-7.70 (m, 2H), 7.65 (dd, 1H), 7.57 (d, 1H), 6.65 (d, 1H)

### Step 3) Preparation of N-(4-(4-methylpiperazin-1-yl)phenyl)-5-(3-nitrophenoxy)-[1,2,4]triazolo[1,5-a]pyridin-2-amine

After 2-iodo-5-(3-nitrophenoxy)-[1,2,4]triazolo[1,5-a]pyridine (100 mg, 0.3 mmol) was dissolved in 1,4-dioxane (1.2 mL), 4-(4-methylpiperazin-1-yl)aniline (60.1 mg, 0.3 mmol), Pd₂(dba)₃ (11.9 mg, 0.01 mmol), Xantphos (15.0 mg, 0.03 mmol) and cesium carbonate (170.4 mg, 0.5 mmol) were added thereto. The reaction mixture was heated at 100°C for 16 hours, and then the solvent was removed under reduced pressure. The residue was separated by column chromatography to obtain N-(4-(4-methylpiperazin-1-yl)phenyl)-5-(3-nitrophenoxy)-[1,2,4]triazolo[1,5-a]pyridin-2-amine (64.0 mg, yield: 54.9 %).

¹H NMR (500MHz, CD₃OD) δ 8.10 (d, 1H), 8.01 (s, 1H), 7.57 (t, 1H), 7.46-7.38 (m, 4H), 7.30 (d, 1H), 6.87 (d, 2H), 6.37 (d, 1H), 3.14-3.12 (m, 4H), 2.62-2.60 (m, 4H), 2.35 (s, 3H)

### Step 4) Preparation of 5-(3-aminophenoxy)-N-(4-(4-methylpiperazin-1-yl)phenyl)-[1,2,4]triazolo[1,5-a]pyridin-2-amine

After N-(4-(4-methylpiperazin-1-yl)phenyl)-5-(3-nitrophenoxy)-[1,2,4]triazolo[1,5-a]pyridin-2-amine (63.0 mg, 0.14 mmol) was dissolved in methanol (2.0 mL) and chloroform (0.8 mL), 10% Pd/C (15.0 mg, 0.01 mmol) was added thereto. The reaction mixture was stirred under hydrogen for 4 hours. After completion of the reaction, the mixture was filtered through celite and the solvent was removed under reduced pressure. The residue was separated by column chromatography to obtain 5-(3-aminophenoxy)-N-(4-(4-methylpiperazin-1-yl)phenyl)-[1,2,4]triazolo[1,5-a]pyridin-2-amine (30.0 mg, yield: 51.1%).

¹H NMR (500MHz, CD₃OD) δ 7.54-7.47 (m, 3H), 7.15-7.11 (m, 2H), 6.99-6.98 (m, 2H), 6.62-6.25 (m, 3H), 6.25-6.24 (m, 1H), 3.40-3.20 (m, 8H)

### Step 5) Preparation of N-(3-(2-(4-(4-methylpiperazin-1-yl)phenylamino)-[1,2,4]triazolo[1,5-a]pyridin-5-yloxy)phenyl)acrylamide

After 5-(3-aminophenoxy)-N-(4-(4-methylpiperazin-1-yl)phenyl)-[1,2,4]triazolo[1,5-a]pyridin-2-amine (30.0 mg, 0.07 mmol) was suspended in dichloromethane (0.5 mL), triethylamine (12.1 µL, 0.09 mmol) and acryloyl chloride (6.5 µL, 0.08 mmol) were added dropwise thereto. The reaction mixture was stirred at room temperature for 1 hour and then concentrated under reduced pressure. The residue was separated by column chromatography to obtain N-(3-(2-(4-(4-methylpiperazin-1-yl)phenylamino)-[1,2,4]triazolo[1,5-a]pyridin-5-yloxy)phenyl)acrylamide (7.0 mg, yield: 20.6%).

¹H NMR (500MHz, CD₃OD) δ 7.70 (1H), 7.53-7.50 (m, 2H), 7.45 (d, 2H), 7.40 (t, 1H), 7.20 (d, 1H), 6.95-6.90 (m, 3H), 6.44-6.43 (m, 3H), 5.77 (dd, 1H), 3.11 (t, 4H), 2.64 (t, 4H), 2.36 (s, 1H)

### Example 2: Preparation of N-(3-(2-(1-methyl-1H-pyrazol-4-ylamino)-[1,2,4]triazolo[1,5-a]pyridin-5-yloxy)phenyl)acrylamide

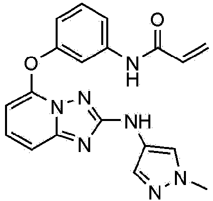

A title compound (9.6 mg, yield 20.0%) was obtained in the same manner as in Example 1, except that in Example 1, 1-methyl-1H-pyrazol-4-amine was used instead of 4-(4-methylpiperazin-1-yl)aniline.

¹H NMR (500MHz, CD₃OD) δ 7.69 (d, 2H), 7.54-7.48 (m, 1H), 7.44-7.40 (m, 1H), 7.20 (1, H), 6.94 (dd, 1H), 6.43-6.32 (m, 3H), 5.76 (dd, 1H), 3.81 (s, 1H)

### Example 3: Preparation of N-(3-(2-(benzo[d]thiazol-6-ylamino)-[1,2,4]triazolo[1,5-a]pyridin-5-yloxy)phenyl)acrylamide

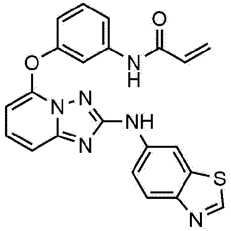

### Step 1) Preparation of N-(3-hydroxyphenyl)acrylamide

After 3-aminophenol (500.0 mg, 4.6 mmol) and sodium bicarbonate (577.8 mg, 6.9 mmol) were dissolved in tetrahydrofuran (10 mL), acryloyl chloride (415.0 mg, 4.6 mmol) was added thereto. The mixture was stirred at room temperature for 12 hours, and then distilled water was added thereto. The organic layer was separated, treated with magnesium sulfate, filtered, and then concentrated under reduced pressure. N-(3-hydroxyphenyl)acrylamide (730.0 mg, yield: 97.6%) was obtained without further purification.

¹H NMR (500MHz, CD₃OD) δ 7.24 (s, 1H), 7.11 (t, 1H), 7.00 (d, 1H), 6.54 (dd, 1H), 6.44-6.39 (m, 1H), 6.33 (dd, 1H), 5.74 (dd, 1H)

### Step 2) Preparation of N-(3-(2-amino-[1,2,4]triazolo[1,5-a]pyridin-5-yloxy)phenyl)acrylamide

After 5-bromo-[1,2,4 triazolo[1,5-a]pyridin-2-amine (435.0 mg, 2.0 mmol) and N-(3-hydroxyphenyl)acrylamide (499.8 mg, 3.1 mmol) were dissolved in N,N-dimethylformamide (5.0 mL), cesium carbonate (1300.0 mg, 4.08 mmol) was added thereto. The mixture was stirred at 100°C for 3 hours and then cooled. Distilled water was added thereto and the solution was stirred for 10 minutes. The organic layer was separated, treated with magnesium sulfate, filtered, and then concentrated under reduced pressure. The residue was separated by column chromatography to obtain N-(3-(2-amino-[1,2,4]triazolo[1,5-a]pyridin-5-yloxy)phenyl)acrylamide (200.0 mg, yield: 33.2%).

¹H NMR (500MHz, CD₃OD) δ 7.69 (s, 1H), 7.52-7.47 (m, 2H), 7.42 (t, 1H), 7.13 (d, 1H), 6.97 (dd, 1H), 6.43-6.33 (m, 2H), 6.29 (d, 1H), 5.77 (dd, 1H)

### Step 3) Preparation of N-(3-(2-iodo-[1,2,4]triazolo[1,5-a]pyridin-5-yloxy)phenyl)acrylamide

After N-(3-(2-amino-[1,2,4]triazolo[1,5-a]pyridin-5-yloxy)phenyl)acrylamide (180.0 mg, 0.6 mmol) and toluenesulfonic acid (347.8 mg , 1.8 mmol) were dissolved in acetonitrile (6.0 mL), a solution of potassium iodide (252.8 mg, 1.5 mmol) and sodium nitrite (84.1 mg, 1.2 mmol) in distilled water (0.5 mL) was added dropwise thereto. The mixture was stirred at room temperature for 16 hours and then distilled water was added thereto. The organic layer was separated, treated with magnesium sulfate, filtered, and then concentrated under reduced pressure. The residue was separated by column chromatography to obtain N-(3-(2-iodo-[1,2,4]triazolo[1,5-a]pyridin-5-yloxy)phenyl)acrylamide (110.0 mg, yield: 44.4%).

¹H NMR (500MHz, CD₃OD) δ 7.75 (s, 1H), 7.67 (t, 1H), 7.53 (d, 1H), 7.48-7.45 (m, 2H), 7.03 (dd, 1H), 6.47 (d, 1H), 6.44-6.33 (m, 2H), 5.78 (dd, 1H)

### Step 4) Preparation of N-(3-(2-(benzo[d]thiazol-6-ylamino)-[1,2,4]triazolo[1,5-a]pyridin-5-yloxy)phenyl)acrylamide

After N-(3-(2-iodo-[1,2,4]triazolo[1,5-a]pyridin-5-yloxy)phenyl)acrylamide (30.0 mg, 0.07 mmol) and benzo[d]thiazol-6-amine (11.1 mg, 0.07 mmol) were dissolved in 1.4-dioxane (1.0 mL), Pd₂(dba)₃ (3.38 mg, 0.004 mmol), Xantphos (4.3 mg, 0.007 mmol) and cesium carbonate (48.1 mg, 0.15 mmol) were added thereto and the reaction was carried out with microwave (120 °C, 10 min, Normal). Distilled water was added thereto and the solution was stirred for 10 minutes. The organic layer was separated, treated with magnesium sulfate, filtered, and then concentrated under reduced pressure. The residue was separated by column chromatography to obtain N-(3-(2-(benzo[d]thiazol-6-ylamino)-[1,2,4]triazolo[1,5-a]pyridin-5-yloxy)phenyl)acrylamide ( 5.0 mg, yield 15.8%).

¹H NMR (500MHz, CD₃OD) δ 8.46 (m, 1H), 7.91 (d, 1H), 7.73 (s, 1H), 7.62-7.51 (m, 3H), 7.46 (t, 1H), 7.31 (d, 1H), 6.99 (dd, 1H), 6.52 (d, 1H), 6.44-6.33 (m, 2H), 5.77 (dd, 1H)

### Example 4: Preparation of N-(3-(2-(4-morpholinophenylamino)-[1,2,4]triazolo[1,5-a]pyridin-5-yloxy)phenyl)acrylamide

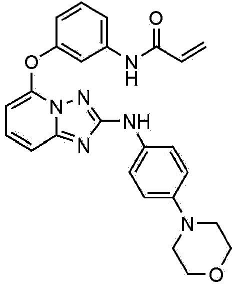

A title compound (6.2 mg, yield: 18.4%) was obtained in the same manner as in Example 3, except that in Example 3, 4-morpholinoaniline was used instead of benzo[d]thiazol-6-amine.

¹H NMR (500MHz, CD₃OD) δ 7.71 (S, 1H), 7.54 (t, 2H), 7.47-7.37 (m, 3H), 7.22 (d, 1H), 6.96-6.91 (m, 3H), 6.44-6.34 (m, 3H), 5.77 (dd, 1H), 3.82 (t, 4H), 3.05 (t, 4H)

### Example 5: Preparation of N-(3-(2-(3-phenoxyphenylamino)-[1,2,4]triazolo[1,5-a]pyridin-5-yloxy)phenyl)acrylamide

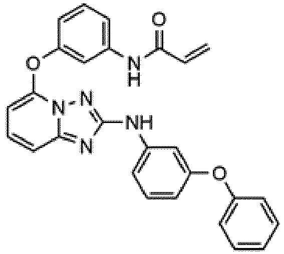

A title compound (5.5 mg, yield: 16.7%) was obtained in the same manner as in Example 3, except that in Example 3, 3-phenoxyaniline was used instead of benzo[d]thiazol-6-amine.

¹H NMR (500MHz, CD₃OD) δ7.68 (s, 1H), 7.55 (t, 1H), 7.50-7.48 (m, 1H), 7.43-7.39 (m, 2H), 7.33-7.29 (m, 3H), 7.25-7.20 (m, 2H), 7.05-7.00 (m, 3H), 6.96 (dd, 1H), 6.52 (dd, 1H), 6.42-6.33 (m, 3H), 5.77 (dd, 1H)

### Example 6: Preparation of N-(3-(2-(4-(dimethylamino)phenylamino)-[1,2,4]triazolo[1,5-a]pyridin-5-yloxy)phenyl)acrylamide

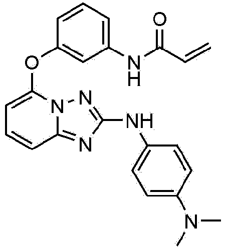

A title compound (4.5 mg, yield: 14.7%) was obtained in the same manner as in Example 3, except that in Example 3, N¹,N¹-dimethylbenzene-1,4-diamine was used instead of benzo[d]thiazol-6-amine.

¹H NMR (500MHz, CD₃OD) δ 7.70 (s, 1H), 7.54-7.51 (m, 2H), 7.44-7.40 (m, 3H), 7.20 (d, 1H), 6.99 (dd, 1H), 6.81 (d, 1H), 6.41-6.37 (m, 3H), 5.78 (dd, 1H), 2.85 (s, 6H)

### Example 7: Preparation of N-(3-(2-(3-(5-methyl-1H-tetrazol-1-yl)phenylamino)-[1,2,4]triazolo[1,5-a]pyridin-5-yloxy)phenyl)acrylamide

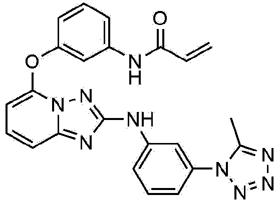

A title compound (4.0 mg, yield: 14.3%) was obtained in the same manner as in Example 3, except that in Example 3, 3-(5-methyl-1H-tetrazol-1-yl)aniline was used instead of benzo[d]thiazol-6-amine.

¹H NMR (500MHz, CD₃OD) δ8.09 (s, 1H), 7.65-7.63 (m, 2H), 7.59 (t, 1H), 7.50 (t, 1H), 7.42 (m, 1H), 7.34 (q, 2H), 7.12 (dd, 1H), 6.93 (dd, 1H), 6.51 (d, 1H), 6.42-6.31 (m, 2H), 5.76 (dd, 1H), 2.60 (s, 3H)

### Example 8: Preparation of N-(3-(2-(1-(difluoromethyl)-1H-pyrazol-4-ylamino)-[1,2,4]triazolo[1,5-a]pyridin-5-yloxy)phenyl)acrylamide

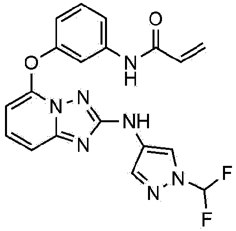

A title compound (5.7 mg, yield: 22.5%) was obtained in the same manner as in Example 3, except that in Example 3, 1-(difluoromethyl)-1H-pyrazol-4-amine was used instead of benzo[d]thiazol-6-amine.

¹H NMR (500MHz, CD₃OD) δ 8.09 (s, 1H), 7.70 (s, 2H), 7.56 (t, 1H), 7.48-7.46 (m, 1H), 7.41 (t, 1H), 7.25 (t, 1H), 6.95 (dd, 1H), 6.48 (d, 1H), 6.42-6.31 (m, 2H), 5.76 (dd, 1H), 2.00 (s, 1H)

### Example 9: Preparation of N-(3-(2-(3-(3,5-dimethyl-1H-pyrazol-1-yl)phenylamino)-[1,2,4]triazolo[1,5-a]pyridin-5-yloxy)phenyl)acrylamide

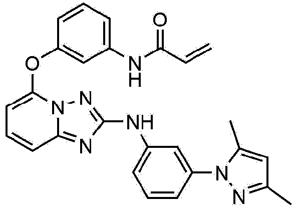

A title compound (0.8 mg, yield: 2.8%) was obtained in the same manner as in Example 3, except that in Example 3, 3-(3,5-dimethyl-1H-pyrazol-1-yl)aniline was used instead of benzo[d]thiazol-6-amine.

¹H NMR (500MHz, CD₃OD) δ 7.91-7.88 (m, 1H), 7.68-7.63 (m, 1H), 7.57 (t, 1H), 7.52-7.51 (m, 1H), 7.47-7.46 (m, 1H), 7.39-7.34 (m, 2H), 7.29 (d, 1H), 6.97-6.92 (m, 2H), 6.48 (d, 1H), 6.42-6.32 (m, 2H), 6.03 (s, 1H), 5.77 (dd, 1H), 2.28 (s, 3H), 2.24 (s, 3H)

### Example 10: Preparation of N-(3-(2-(4-(3,5-dimethyl-1H-pyrazol-1-yl)phenylamino)-[1,2,4]triazolo[1,5-a]pyridin-5-yloxy)phenyl)acrylamide

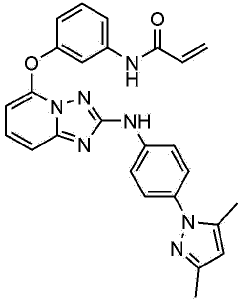

A title compound (1.8 mg, yield: 6.3%) was obtained in the same manner as in Example 3, except that in Example 3, 4-(3,5-dimethyl-1H-pyrazol-1-yl)aniline was used instead of benzo[d]thiazol-6-amine.

¹H NMR (500MHz, CD₃OD) δ 7.73-7.71 (m, 3H), 7.58 (t, 1H), 7.50-7.48 (m, 1H), 7.42 (t, 1H), 7.29-7.27 (m, 3H), 6.97 (dd, 1H), 6.48 (d, 1H), 6.42-6.31 (m, 2H), 6.02 (s, 1H), 5.75 (dd, 1H), 2.22 (s, 3H), 2.22 (s, 3H)

### Example 11: Preparation of N-(3-(2-(2-chlorobenzo[d]thiazol-6-ylamino)-[1,2,4]triazolo[1,5-a]pyridin-5-yloxy)phenyl)acrylamide

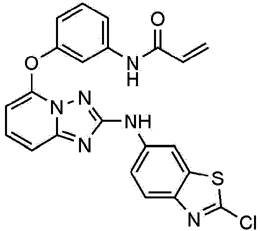

A title compound (3.9 mg, yield: 13.7%) was obtained in the same manner as in Example 3, except that in Example 3, 2-chlorobenzo[d]thiazol-6-amine was used instead of benzo[d]thiazol-6-amine.

¹H NMR (500MHz, CD₃OD) δ 8.32-8.32 (m, 1H), 7.57-7.74 (m, 1H), 7.59 (t, 1H), 7.51-7.30 (m, 5H), 6.95 (dd, 1H), 6.53 (d, 1H), 6.42-6.33 (m, 2H), 5.77 (dd, 1H)

### Example 12: Preparation of N-(3-(2-(benzo[d]thiazol-5-ylamino)-[1,2,4]triazolo[1,5-a]pyridin-5-yloxy)phenyl)acrylamide

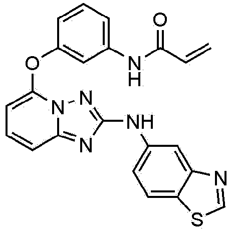

A title compound (6.2 mg, yield: 23.5%) was obtained in the same manner as in Example 3, except that in Example 3, benzo[d]thiazol-5-amine was used instead of benzo[d]thiazol-6-amine.

¹H NMR (500MHz, CD₃OD) δ 9.17 (s, 1H), 8.64 (dd, 1H), 7.91 (d, 1H), 7.74 (s, 1H), 7.65 (dd, 1H), 7.55 (m, 2H), 7.45 (t, 1H), 7.27 (d, 1H), 7.03 (dd, 1H), 6.44-6.33 (m, 3H), 5.77 (dd, 1H)

### Example 13: Preparation of N-(3-(2-(4-phenoxyphenylamino)-[1,2,4]triazolo[1,5-a]pyridin-5-yloxy)phenyl)acrylamide

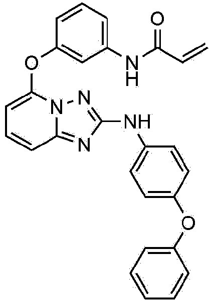

A title compound (5.7 mg, yield: 20.0%) was obtained in the same manner as in Example 3, except that in Example 3, 4-phenoxyaniline was used instead of benzo[d]thiazol-6-amine.

¹H NMR (500MHz, CD₃OD) δ 7.72 (s, 1H), 7.58-7.54 (m, 3H), 7.50-7.48 (m, 1H), 7.42 (t, 1H), 7.29 (t, 2H), 7.25 (d, 1H), 7.03 (t, 1H), 6.98-6.92 (m, 5H), 6.44-6.34 (m, 3H), 5.75 (dd, 1H)

### Example 14: Preparation of N-(3-(2-(1-(3-methoxybenzyl)-1H-pyrazol-4-ylamino)-[1,2,4]triazolo[1,5-a]pyridin-5-yloxy)phenyl)acrylamide

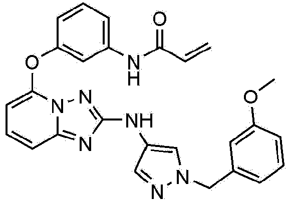

A title compound (3.5 mg, yield: 11.8%) was obtained in the same manner as in Example 3, except that in Example 3, 1-(3-methoxybenzyl)-1H-pyrazol-4-amine was used instead of benzo[d]thiazol-6-amine.

¹H NMR (500MHz, CD₃OD) δ 7.72 (s, 1H), 7.58-7.54 (m, 3H), 7.50-7.48 (m, 1H), 7.42 (t, 1H), 7.29 (t, 2H), 7.25 (d, 1H), 7.03 (t, 1H), 6.98-6.92 (m, 5H), 6.44-6.34 (m, 3H), 5.75 (dd, 1H)

### Example 15: Preparation of N-(3-(2-(4-(2-(diethylamino)ethoxy)phenylamino)-[1,2,4]triazolo[1,5-a]pyridin-5-yloxy)phenyl)acrylamide

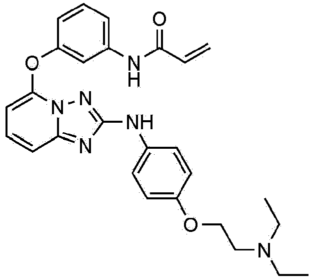

A title compound (4.6 mg, yield: 15.4%) was obtained in the same manner as in Example 3, except that in Example 3, 4-(2-(diethylamino)ethoxy)aniline was used instead of benzo[d]thiazol-6-amine.

¹H NMR (500MHz, CD₃OD) δ 7.71 (s, 1H), 7.56-7.49 (m, 5H), 7.43 (t, 1H), 7.22 (d, 1H), 6.97 (dd, 1H), 6.90 (d, 2H), 6.44-6.33 (m, 3H), 5.78 (dd, 1H), 4.15 (t, 2H), 3.16-3.14 (m, 2H), 2.92-2.91 (m, 4H), 1.96 (t, 6H)

### Example 16: Preparation of N-(3-(2-(4-((dimethylamino)methyl)phenylamino)-[1,2,4]triazolo[1,5-a]pyridin-5-yloxy)phenyl)acrylamide

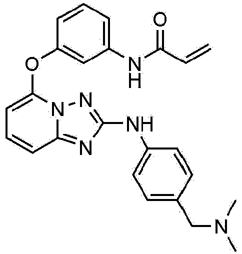

A title compound (3.9 mg, yield: 14.8%) was obtained in the same manner as in Example 3, except that in Example 3, 4-((dimethylamino)methyl)aniline was used instead of benzo[d]thiazol-6-amine.

¹H NMR (500MHz, CD₃OD) δ7.71 (s, 1H), 7.57-7.50 (m, 4H), 7.43 (t, 1H), 7.26 (d, 1H), 7.21 (d, 2H), 6.98-6.97 (m, 1H), 6.45-6.36 (m, 3H), 5.77 (dd, 1H), 3.47 (s, 2H), 2.26 (s, 6H)

### Example 17: Preparation of N-(3-(2-(4-(morpholine-4-carbonyl)phenylamino)-[1,2,4]triazolo[1,5-a]pyridin-5-yloxy)phenyl)acrylamide

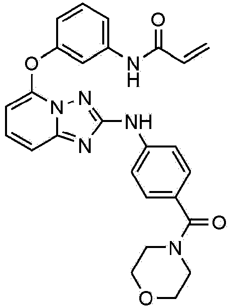

A title compound (7.7 mg, yield: 25.8%) was obtained in the same manner as in Example 3, except that in Example 3, (4-aminophenyl)(morpholino)methanone was used instead of benzo[d]thiazol-6-amine.

¹H NMR (500MHz, CD₃OD) δ 7.73 (s, 1H), 7.68 (d, 2H), 7.58 (t, 1H), 7.54-7.53 (m, 1H), 7.44-7.40 (m, 1H), 7.37 (d, 2H), 7.29 (d, 1H), 6.97 (dd, 1H), 6.47 (d, 1H), 6.44-6.33 (m, 2H), 5.78 (dd, 1H), 3.68 (m, 8H)

### Example 18: Preparation of N-(3-(2-(4-(piperidin-1-ylsulfonyl)phenylamino)-[1,2,4]triazolo[1,5-a]pyridin-5-yloxy)phenyl)acrylamide

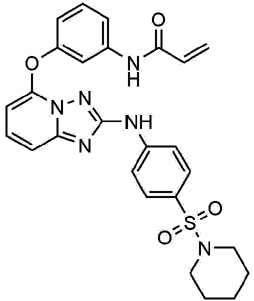

A title compound (6.4 mg, yield: 20.1%) was obtained in the same manner as in Example 3, except that in Example 3, 4-(piperidin-1-ylsulfonyl)aniline was used instead of benzo[d]thiazol-6-amine.

¹H NMR (500MHz, CD₃OD) δ 7.79-7.77 (m, 3H), 7.63-7.59 (m, 3H), 7.48 (d, 1H), 7.42 (t, 1H), 7.32 (d, 1H), 6.96 (dd, 1H), 6.52 (d, 1H), 6.45-6.34 (m, 2H), 5.78 (dd, 1H), 2.95-2.92 (m, 4H), 1.64-1.60 (m, 4H), 1.42 (m, 2H)

### Example 19: Preparation of N-(3-(2-(1-(2-morpholinoethyl)-1H-pyrazol-4-ylamino)-[1,2,4]triazolo[1,5-a]pyridin-5-yloxy)phenyl)acrylamide

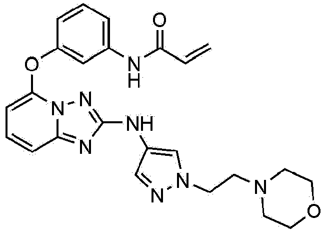

A title compound (3.5 mg, yield: 12.0%) was obtained in the same manner as in Example 3, except that in Example 3, 1-(2-morpholinoethyl)-1H-pyrazol-4-amine was used instead of benzo[d]thiazol-6-amine.

¹H NMR (500MHz, CD₃OD) δ 7.86 (s, 1H), 7.70 (s, 1H), 7.56-7.43 (m, 4H), 7.21 (d, 1H), 6.94 (d, 1H), 6.43-6.36 (m, 3H), 7.77 (dd, 1H), 4.25 (t, 2H), 3.75-3.60 (m, 4H), 2.90 (m, 2H), 2.65-2.50 (m, 4H)

### Example 20: Preparation of N-(3-(2-(4-(4-methylpiperazin-1-yl)phenylamino)-[1,2,4]triazolo[1,5-a]pyridin-5-yloxy)phenyl)but-2-ynamide

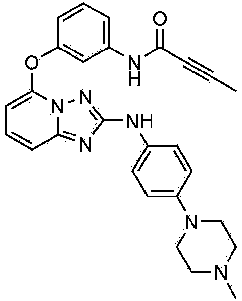

A title compound (5.3 mg, yield: 17.9%) was obtained in the same manner as in Example 1, except that in Example 1, the step 5 was carred out as follows,.

After 5-(3-aminophenoxy)-N-(4-(4-methylpiperazin-1-yl)phenyl)-[1,2,4]triazolo[1,5-a]pyridin-2-amine (20.0 mg, 0.05 mmol) and but-2-inoic acid (4.86 mg, 0.06 mmol) were dissolved in tetrahydrofuran (0.5 mL), 1-[bis(dimethylamino)methylene]-1H-1,2,3-triazolo[4,5-b]pyridinium 3-oxidehexafluorophosphate (22.0 mg, 0.06 mmol) and N,N-diisopropylethylamine (25.0 µL, 0.15 mmol) were added thereto. The reaction mixutre was stirred at room temperature for 20 hours and then concentrated under reduced pressure. The residue was separated by column chromatography to obtain N-(3-(2-(4-(4-methylpiperazin-1-yl)phenylamino)-[1,2,4]triazolo[1,5-a]pyridin-5-yloxy)phenyl)but-2-ynamide.

¹H NMR (500MHz, CD₃OD) δ 7.60 (s, 1H), 7.51 (t, 1H), 7.45-7.44 (m, 3H), 7.38 (t, 1H), 7.20 (d, 1H), 6.92 (d, 3H), 6.38 (d, 1H), 3.20-3.10 (m, 4H), 2.75-2.65 (m, 4H), 2.42 (s, 3H), 2.02 (s, 3H)

### Example 21: Preparation of N-(3-(2-(6-morpholinopyridin-3-ylamino)-[1,2,4]triazolo[1,5-a]pyridin-5-yloxy)phenyl)acrylamide

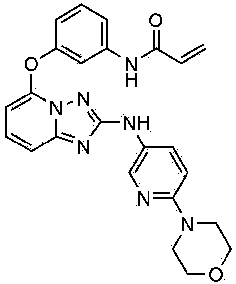

A title compound (7.7 mg, yield: 27.4%) was obtained in the same manner as in Example 3, except that in Example 3, 6-morpholinopyridin-3-amine was used instead of benzo[d]thiazol-6-amine.

¹H NMR (500MHz, CD₃OD) δ 8.39 (d, 1H), 7.92 (dd, 1H), 7.70 (s, 1H), 7.56-7.51 (m, 2H), 7.42 (t, 1H), 6.95 (dd, 1H), 6.80 (d, 1H), 6.44-6.33 (m, 3H), 5.78 (dd, 1H), 3.78 (t, 4H), 3.36 (t, 4H)

### Example 22: Preparation of N-(3-(2-(6-chlorobenzo[d]thiazol-2-ylamino)-[1,2,4]triazolo[1,5-a]pyridin-5-yloxy)phenyl)acrylamide

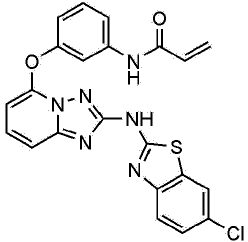

A title compound (2.5 mg, yield: 11.0%) was obtained in the same manner as in Example 3, except that in Example 3, 6-chlorobenzo[d]thiazol-2-amine was used instead of benzo[d]thiazol-6-amine.

¹H NMR (500MHz, CD₃OD) δ 7.75 (s, 1H), 7.67 (t, 1H), 7.62 (d, 1H), 7.53-7.52 (m, 1H), 7.48-7.45 (m, 2H), 7.31 (d, 1H), 7.24 (dd, 1H), 7.03 (dd, 1H), 6.47 (d, 1H), 6.41-6.37 (m, 2H), 5.78 (dd, 1H)

### Example 23: Preparation of N-(3-(2-(naphthalen-1-ylamino)-[1,2,4]triazolo[1,5-a]pyridin-5-yloxy)phenyl)acrylamide

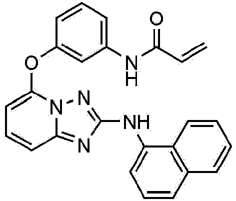

A title compound (6.2 mg, yield: 29.9%) was obtained in the same manner as in Example 3, except that in Example 3, naphthalen-1-amine was used instead of benzo[d]thiazol-6-amine.

¹H NMR (500MHz, CD₃OD) δ 8.19-8.18 (m, 1H), 7.96 (d, 1H), 7.88-7.86 (m, 1H), 7.71 (s, 1H), 7.61 (d, 1H), 7.56 (t, 1H), 7.53-7.48 (m, 3H), 7.44 (td, 2H), 7.25 (d, 1H), 7.49 (dd, 1H), 6.44-6.33 (m, 3H), 5.78 (dd, 1H)

### Example 24: Preparation of N-(3-(2-(quinolin-3-ylamino)-[1,2,4]triazolo[1,5-a]pyridin-5-yloxy)phenyl)acrylamide

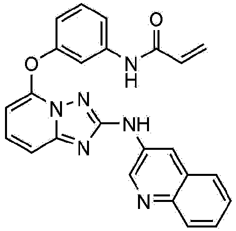

A title compound (5.2 mg, yield: 25.0%) was obtained in the same manner as in Example 3, except that in Example 3, quinolin-3-amine was used instead of benzo[d]thiazol-6-amine.

¹H NMR (500MHz, CD₃OD) δ 8.90 (d, 1H), 8.69 (d, 1H), 7.91 (d, 1H), 7.78-7.75 (m, 2H), 7.64 (t, 1H), 7.57-7.50 (m, 3H), 7.45 (t, 1H), 7.37 (d, 1H), 6.97 (dd, 1H), 6.58 (d, 1H), 6.46-6.35 (m, 2H), 5.78 (dd, 1H)

### Example 25: Preparation of N-(3-(2-(3-methylisoxazolo[5,4-b]pyridin-5-ylamino)-[1,2,4]triazolo[1,5-a]pyridin-5-yloxy)phenyl)acrylamide

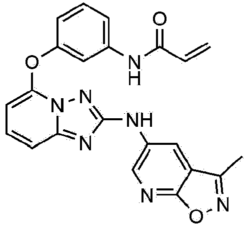

A title compound (3.2 mg, yield: 15.2%) was obtained in the same manner as in Example 3, except that in Example 3, 3-methylisoxazolo[5,4-b]-pyridin-5-amine was used instead of benzo[d]thiazol-6-amine.

¹H NMR (500MHz, CD₃OD) δ 8.66 (dd, 2H), 7.73 (s, 1H), 7.64-7.61 (m, 1H), 7.48-7.40 (m, 2H), 7.36-7.34 (m, 1H), 6.96-6.94 (m, 1H), 6.54 (d, 1H), 6.43-6.33 (m, 2H), 5.76 (dd, 1H), 2.53 (s, 3H)

### Example 26: Preparation of N-(3-(2-(1-methyl-1H-imidazo[4,5-b]pyridin-6-ylamino)-[1,2,4]triazolo[1,5-a]pyridin-5-yloxy)phenyl)acrylamide

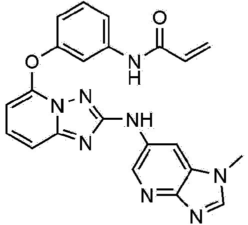

A title compound (3.0 mg, yield: 14.3%) was obtained in the same manner as in Example 3, except that in Example 3, 1-methyl-1H-imidazo[4,5-b]pyridin-6-amine was used instead of benzo[d]thiazol-6-amine.

¹H NMR (500MHz, CD₃OD) δ 8.72 (d, 1H), 8.68 (d, 1H), 8.26 (s, 1H), 7.53 (t, 2H), 7.47-7.34 (m, 2H), 7.21-7.12 (m, 2H), 6.64 (dd, 1H), 6.59-6.58 (m, 1H), 6.52 (dd, 1H), 6.29 (d, 1H), 3.92 (s, 3H)

### Example 27: Preparation of N-(4-fluoro-3-(2-(4-morpholinophenylamino)-[1,2,4]triazolo[1,5-a]pyridin-5-yloxy)phenyl)acrylamide

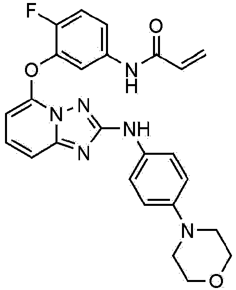

A title compound (8.3 mg, yield: 37.1%) was obtained in the same manner as in Example 3, except that in Example 3, 5-amino-2-fluorophenol was used instead of 3-amino phenol, and 4-morpholinoaniline was used instead of benzo[d]thiazol-6-amine.

¹H NMR (500MHz, CD₃OD) δ 7.78 (dd, 1H), 7.58-7.47 (m, 4H), 7.32 (t, 1H), 7.22 (d, 1H), 6.93 (d, 2H), 6.41-6.35 (m, 3H), 5.77 (dd, 1H), 3.82 (t, 4H), 3.06 (t, 4H)

### Example 28: Preparation of N-(3-(2-(benzo[d]thiazol-6-ylamino)-[1,2,4]triazolo[1,5-a]pyridin-5-yloxy)-4-fluorophenyl)acrylamide

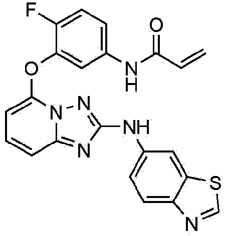

A title compound (5.8 mg, yield: 27.6%) was obtained in the same manner as in Example 3, except that in Example 3, 5-amino-2-fluorophenol was used instead of 3-aminophenol.

¹H NMR (500MHz, CD₃OD) δ 8.51 (s, 1H), 7.94 (t, 1H), 7.81 (dd, 1H), 7.64-7.52 (m, 4H), 7.39-7.32 (m, 2H), 6.52-6.50 (m, 1H), 6.38-6.34 (m, 2H), 5.76 (dd, 1H)

### Example 29: Preparation of N-(4-fluoro-3-(2-(4-(4-methylpiperazin-1-yl)phenylamino)-[1,2,4]triazolo[1,5-a]pyridin-5-yloxy)phenyl)acrylamide

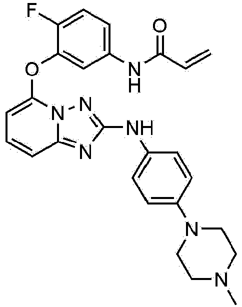

A title compound (3.7 mg, yield: 16.1%) was obtained in the same manner as in Example 3, except that in Example 3, 5-amino-2-fluorophenol was used instead of 3-aminophenol, and 4-(4-methylpiperazin-1-yl)aniline was used instead of benzo[d]thiazol-6-amine.

¹H NMR (500MHz, CD₃OD) δ 7.79 (dd, 1H), 7.58-7.52 (m, 2H), 7.48 (d, 2H), 7.32 (t, 1H), 7.23 (d, 1H), 6.95 (d, 2H), 6.41-6.33 (m, 3H), 5.77 (s, 1H), 3.16 (m, 4H), 2.75 (m, 4H), 2.44 (s, 3H)

### Example 30: Preparation of N-(4-fluoro-3-(2-(1-methyl-1H-pyrazol-4-ylamino)-[1,2,4]triazolo[1,5-a]pyridin-5-yloxy)phenyl)acrylamide

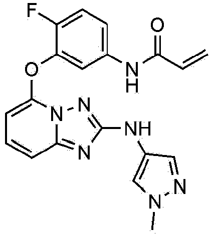

A title compound (0.9 mg, yield: 3.9%) was obtained in the same manner as in Example 3, except that in Example 3, 5-amino-2-fluorophenol was used instead of 3-amino phenol, and 1-methyl-1H-pyrazol-4-amine was used instead of benzo[d]thiazol-6-amine.

¹H NMR (500MHz, CD₃OD) δ 7.80-7.78 (m, 1H), 7.76-7.75 (m, 1H), 7.68-7.66 (m, 1H), 7.55-7.52 (m, 2H), 7.47-7.46 (m, 1H), 7.36-7.32 (m, 1H), 7.23-7.21 (m, 1H), 6.41-6.39 (m, 1H), 6.37-6.35 (m, 1H), 5.78-5.76 (m, 1H), 3.84 (s, 3H)

### Example 31: Preparation of N-(3-(2-(1-(difluoromethyl)-1H-pyrazol-4-ylamino)-[1,2,4]triazolo[1,5-a]pyridin-5-yloxy)-4-fluorophenyl)acrylamide

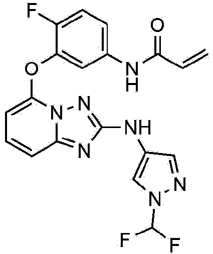

A title compound (1.4 mg, yield: 5.5%) was obtained in the same manner as in Example 3, except that in Example 3, 5-amino-2-fluorophenol was used instead of 3-amino phenol, and 1-(difluoromethyl)-1H-pyrazol-4-amine was used instead of benzo[d]thiazol-6-amine.

¹H NMR (500MHz, CD₃OD) δ 8.14-8.13 (m, 1H), 7.79-7.77 (m, 1H), 7.59 (s, 1H), 7.58-7.56 (m, 1H), 7.52-7.49 (m, 1H), 7.35-7.25 (m, 2H), 6.48-6.46 (m, 1H), 6.36-6.31 (m, 2H), 5.77-5.75 (m, 1H)

### Experimental Example 1: JAK 3 Inhibitory Activity Test

The inhibitory activities against JAK3were measured for the compounds prepared in the above Examples through in vitro analysis on the ADP Glow (Glo) platform.

Specifically, the inhibitory activities against JAK3 were measured using a JAK3 kinase assay kit (Promega, V9441), which was purchased from Promega. Recombinant purified human JAK3 was diluted with 1 x kinase reaction buffer (JAK3: 40 mM Tris-Cl, pH 7.5, 20 mM MgCl₂, 0.1 mg/mL BSA and 50 uM DTT and added to a 96 well plate (JAK3: final concentration of 4 ng per reaction). The compounds prepared in the previous Examples were treated so as to be finally a 1% DMSO aqueous solution, and a substrate cocktail containing ATP (JAK3: final concentration of 5 uM) and 0.2 ug/ul of Poly(Glu4, Tyr1) peptide (JAK3 final concentration) in the total 25 uL reactants was added to a 96-well plate to initiate enzymatic reaction. After incubation (30°C) for 1 hour, equivalent volume (25 uL per reaction) of ADP Glo was added and incubated(30°C) for 40 minutes at room temperature. Then, a kinase detection reagent (50 uL per reaction) was added and incubated (30°C) for 30 minutes at room temperature. The kinase activity was measured by chemiluminescence according to the instructions of ADP Glo kinase assay kit, and the inhibitory activity of the compounds according to the present invention was calculated. For the analysis of the results of each compound, Microsoft Excel was used, and IC50 values were calculated by SigmaPlot software. The results are shown in Table 1 below.

**[Table 1]**

| Example No. | JAK3 IC₅₀ (nM) | Example No. | JAK3 IC₅₀ (nM) |
|---|---|---|---|
| 1 | 0.47 | 17 | 0.7 |
| 2 | 1.1 | 18 | 3 |
| 3 | 2.4 | 19 | 5.1 |
| 4 | 0.6 | 20 | 0.6 |
| 5 | 35.8 | 21 | 0.7 |
| 6 | 2.1 | 22 | > 400 |
| 7 | 1.2 | 23 | > 400 |
| 8 | 1.3 | 24 | 15.5 |
| 9 | 10.4 | 25 | 105.2 |
| 10 | 3.6 | 26 | > 80 |
| 11 | 10.3 | 27 | 1.3 |
| 12 | 2.6 | 28 | 1.2 |
| 13 | 9.8 | 29 | 0.7 |
| 14 | 11.1 | 30 | 13.1 |
| 15 | 6.2 | 31 | 18.2 |
| 16 | 0.7 | - | - |

## Claims

1. A compound represented by the following Chemical Formula 1, or a pharmaceutically acceptable salt thereof: 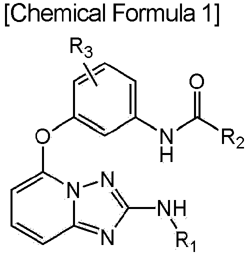 in Chemical Formula 1,
R₁ is C₆₋₁₀ aryl, or C₃₋₁₀ heteroaryl containing 1 to 3 heteroatoms each independently selected from the group consisting of N, O and S,
wherein R₁ is unsubstituted, or substituted with: C₁₋₄alkyl, C₁₋₄alkyl substituted with N(C₁₋₄ alkyl)₂, C₁₋₄alkyl substituted with morpholino, C₁₋₄haloalkyl, halogen, C₁₋₄ alkoxy, C₁₋₄ alkoxy substituted with N(C₁₋₄ alkyl)₂, N(C₁₋₄ alkyl)₂, morpholino, morpholinocarbonyl, phenoxy, piperidinyl, -SO₂-piperidinyl, piperazinyl, piperazinyl substituted with C₁₋₄ alkyl, benzyl, benzyl substituted with C₁₋₄ alkoxy, pyrazolyl, pyrazolyl substituted with one or two C₁₋₄ alkyl, tetrazolyl, or tetrazolyl substituted with C₁₋₄ alkyl,
R₂ is C₁₋₅ alkyl, C₂₋₅ alkenyl, or C₂₋₅ alkynyl,
wherein R₂ is unsubstituted, or substituted with one or two substituents selected from the group consisting of halogen, cyano, C₃₋₆ cycloalkyl and C₁₋₅ alkyl substituted with cyano, and
R₃ is hydrogen, halogen, C₁₋₄ alkyl, C₁₋₄ haloalkyl, or C₁₋₄ alkoxy.

2. The compound or a pharmaceutically acceptable salt thereof according to claim 1, wherein:
the Chemical Formula 1 is represented by the following Chemical Formula 1-1: 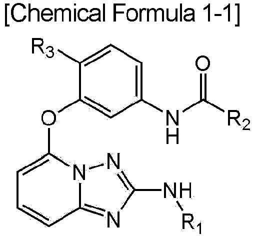 in Chemical Formula 1-1,
R₁ to R₃ are as defined in claim 1.

3. The compound or a pharmaceutically acceptable salt thereof according to claim 1, wherein:
R₁ is benzothiazolyl, imidazo[4,5-b]pyridinyl, isoxazolo[5,4-b]pyridinyl, naphthyl, phenyl, pyrazolyl, pyridinyl, or quinolinyl.

4. The compound or a pharmaceutically acceptable salt thereof according to claim 1, wherein:
R₁ is phenyl,
wherein R₁ is substituted with: C₁₋₄ alkyl substituted with N(C₁₋₄ alkyl)₂, C₁₋₄ alkoxy substituted with N(C₁₋₄ alkyl)₂, N(C₁₋₄ alkyl)₂, morpholino, morpholinocarbonyl, phenoxy, - SO₂-piperidinyl, piperazinyl substituted with C₁₋₄ alkyl, pyrazolyl substituted with two C₁₋₄ alkyl, or tetrazolyl substituted with C₁₋₄ alkyl.

5. The compound or a pharmaceutically acceptable salt thereof according to claim 1, wherein:
R₁ is pyrazolyl,
wherein R₁ is substituted with: C₁₋₄alkyl, C₁₋₄alkyl substituted with morpholino, C₁₋₄ haloalkyl, or benzyl substituted with C₁₋₄ alkoxy.

6. The compound or a pharmaceutically acceptable salt thereof according to claim 1, wherein:
R₁ is unsubstituted, or is benzothiazolyl substituted with halogen;
imidazo[4,5-b]pyridinyl substituted with C₁₋₄ alkyl;
isoxazolo[5,4-b]pyridinyl substituted with C₁₋₄ alkyl;
unsubstituted naphthyl;
pyridinyl substituted with morpholino; or
unsubstituted quinolinyl.

7. The compound or a pharmaceutically acceptable salt thereof according to claim 1, wherein:
R₂ is any one selected from the group consisting of the following:

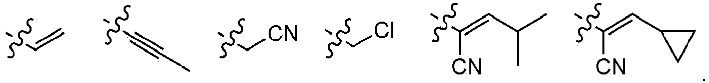

8. The compound or a pharmaceutically acceptable salt thereof according to claim 1, wherein:
R₃ is hydrogen, fluoro, chloro, methyl, trifluoromethyl, or methoxy.

9. The compound or a pharmaceutically acceptable salt thereof according to claim 1, wherein:
the compound represented by Chemical Formula 1 is any one selected from the group consisting of:
1) N-(3-(2-(4-(4-methylpiperazin-1-yl)phenylamino)-[1,2,4]triazolo[1,5-a]pyridin-5-yloxy)phenyl)acrylamide,
2) N-(3-(2-(1-methyl-1H-pyrazol-4-ylamino)-[1,2,4]triazolo[1,5-a]pyridin-5-yloxy)phenyl)acrylamide,
3) N-(3-(2-(benzo[d]thiazol-6-ylamino)-[1,2,4]triazolo[1,5-a]pyridin-5-yloxy)phenyl)acrylamide,
4) N-(3-(2-(4-morpholinophenylamino)-[1,2,4]triazolo[1,5-a]pyridin-5-yloxy)phenyl)acrylamide,
5) N-(3-(2-(3-phenoxyphenylamino)-[1,2,4]triazolo[1,5-a]pyridin-5-yloxy)phenyl)acrylamide,
6) N-(3-(2-(4-(dimethylamino)phenylamino)-[1,2,4]triazolo[1,5-a]pyridin-5-yloxy)phenyl)acrylamide,
7) N-(3-(2-(3-(5-methyl-1H-tetrazol-1-yl)phenylamino)-[1,2,4]triazolo[1,5-a]pyridin-5-yloxy)phenyl)acrylamide,
8) N-(3-(2-(1-(difluoromethyl)-1H-pyrazol-4-ylamino)-[1,2,4]triazolo[1,5-a]pyridin-5-yloxy)phenyl)acrylamide,
9) N-(3-(2-(3-(3,5-dimethyl-1H-pyrazol-1-yl)phenylamino)-[1,2,4]triazolo[1,5-a]pyridin-5-yloxy)phenyl)acrylamide,
10) N-(3-(2-(4-(3,5-dimethyl-1H-pyrazol-1-yl)phenylamino)-[1,2,4]triazolo[1,5-a]pyridin-5-yloxy)phenyl)acrylamide,
11) N-(3-(2-(2-chlorobenzo[d]thiazol-6-ylamino)-[1,2,4]triazolo[1,5-a]pyridin-5-yloxy)phenyl)acrylamide,
12) N-(3-(2-(benzo[d]thiazol-5-ylamino)-[1,2,4]triazolo[1,5-a]pyridin-5-yloxy)phenyl)acrylamide,
13) N-(3-(2-(4-phenoxyphenylamino)-[1,2,4]triazolo[1,5-a]pyridin-5-yloxy)phenyl)acrylamide,
14) N-(3-(2-(1-(3-methoxybenzyl)-1H-pyrazol-4-ylamino)-[1,2,4]triazolo[1,5-a]pyridin-5-yloxy)phenyl)acrylamide,
15) N-(3-(2-(4-(2-(diethylamino)ethoxy)phenylamino)-[1,2,4]triazolo[1,5-a]pyridin-5-yloxy)phenyl)acrylamide,
16) N-(3-(2-(4-((dimethylamino)methyl)phenylamino)-[1,2,4]triazolo[1,5-a]pyridin-5-yloxy)phenyl)acrylamide,
17) N-(3-(2-(4-(morpholine-4-carbonyl)phenylamino)-[1,2,4]triazolo[1,5-a]pyridin-5-yloxy)phenyl)acrylamide,
18) N-(3-(2-(4-(piperidin-1-ylsulfonyl)phenylamino)-[1,2,4]triazolo[1,5-a]pyridin-5-yloxy)phenyl)acrylamide,
19) N-(3-(2-(1-(2-morpholinoethyl)-1H-pyrazol-4-ylamino)-[1,2,4]triazolo[1,5-a]pyridin-5-yloxy)phenyl)acrylamide,
20) N-(3-(2-(4-(4-methylpiperazin-1-yl)phenylamino)-[1,2,4]triazolo[1,5-a]pyridin-5-yloxy)phenyl)but-2-ynamide,
21) N-(3-(2-(6-morpholinopyridin-3-ylamino)-[1,2,4]triazolo[1,5-a]pyridin-5-yloxy)phenyl)acrylamide,
22) N-(3-(2-(6-chlorobenzo[d]thiazol-2-ylamino)-[1,2,4]triazolo[1,5-a]pyridin-5-yloxy)phenyl)acrylamide,
23) N-(3-(2-(naphthalen-1-ylamino)-[1,2,4]triazolo[1,5-a]pyridin-5-yloxy)phenyl)acrylamide,
24) N-(3-(2-(quinolin-3-ylamino)-[1,2,4]triazolo[1,5-a]pyridin-5-yloxy)phenyl)acrylamide,
25) N-(3-(2-(3-methylisoxazolo[5,4-b]pyridin-5-ylamino)-[1,2,4]triazolo[1,5-a]pyridin-5-yloxy)phenyl)acrylamide,
26) N-(3-(2-(1-methyl-1H-imidazo[4,5-b]pyridin-6-ylamino)-[1,2,4]triazolo[1,5-a]pyridin-5-yloxy)phenyl)acrylamide,
27) N-(4-fluoro-3-(2-(4-morpholinophenylamino)-[1,2,4]triazolo[1,5-a]pyridin-5-yloxy)phenyl)acrylamide,
28) N-(3-(2-(benzo[d]thiazol-6-ylamino)-[1,2,4]triazolo[1,5-a]pyridin-5-yloxy)-4-fluorophenyl)acrylamide,
29) N-(4-fluoro-3-(2-(4-(4-methylpiperazin-1-yl)phenylamino)-[1,2,4]triazolo[1,5-a]pyridin-5-yloxy)phenyl)acrylamide,
30) N-(4-fluoro-3-(2-(1-methyl-1H-pyrazol-4-ylamino)-[1,2,4]triazolo[1,5-a]pyridin-5-yloxy)phenyl)acrylamide, and
31) N-(3-(2-(1-(difluoromethyl)-1H-pyrazol-4-ylamino)-[1,2,4]triazolo[1,5-a]pyridin-5-yloxy)-4-fluorophenyl)acrylamide.

10. A pharmaceutical composition for use in the prevention or treatment of inflammatory disease, autoimmune disease, proliferative disease, hyperproliferative disease, immunity mediated disease, cancer or tumor, comprising the compound according to any one of claims 1 to 9 or a pharmaceutically acceptable salt thereof.

## Patentansprüche

1. Verbindung, die durch die folgende chemische Formel 1 dargestellt wird, oder ein pharmazeutisch akzeptables Salz davon: 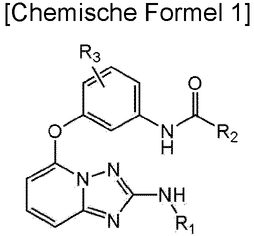 wobei in der chemischen Formel 1
R₁ C₆₋₁₀-Aryl oder C₃₋₁₀-Heteroaryl ist, das 1 bis 3 Heteroatome enthält, die jeweils unabhängig voneinander aus der Gruppe bestehend aus N, O und S ausgewählt sind,
wobei R₁ unsubstituiert ist oder substituiert wird mit: C₁₋₄-Alkyl, C₁₋₄-Alkyl substituiert mit N(C₁₋₄-Alkyl)₂, C₁₋₄-Alkyl substituiert mit Morpholino, C₁₋₄-Halogenalkyl, Halogen, C₁₋₄-Alkoxy, C₁₋₄-Alkoxy substituiert mit N(C₁₋₄-Alkyl)₂, N(C₁₋₄-Alkyl)₂, Morpholino, Morpholinocarbonyl, Phenoxy, Piperidinyl, -SO₂-Piperidinyl, Piperazinyl, Piperazinyl substituiert mit C₁₋₄-Alkyl, Benzyl, Benzyl substituiert mit C₁₋₄-Alkoxy, Pyrazolyl, Pyrazolyl substituiert mit einem oder zwei C₁₋₄-Alkylgruppen, Tetrazolyl oder Tetrazolyl substituiert mit C₁₋₄-Alkyl,
R₂ C₁₋₅-Alkyl, C₂₋₅-Alkenyl oder C₂₋₅-Alkinyl ist,
wobei R₂ unsubstituiert ist oder mit einem oder zwei Substituenten substituiert wird, die aus der Gruppe ausgewählt sind, bestehend aus Halogen, Cyano, C₃₋₆-Cycloalkyl und C₁₋₅-Alkyl, das mit Cyano substituiert ist, und
R₃ Wasserstoff, Halogen, C₁₋₄-Alkyl, C₁₋₄-Halogenalkyl oder C₁₋₄-Alkoxy ist.

2. Verbindung oder ein pharmazeutisch akzeptables Salz davon nach Anspruch 1, wobei:
die Chemische Formel 1 durch folgende Chemische Formel 1-1 dargestellt wird: 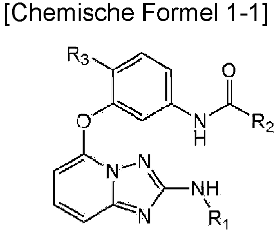 wobei in der chemischen Formel 1-1
R₁ bis R₃ wie in Anspruch 1 definiert sind.

3. Verbindung oder ein pharmazeutisch akzeptables Salz davon nach Anspruch 1, wobei:
R₁ Benzothiazolyl, Imidazo[4,5-b]pyridinyl, Isoxazol[5,4-b]pyridinyl, Naphthyl, Phenyl, Pyrazolyl, Pyridinyl oder Chinolinyl ist.

4. Verbindung oder ein pharmazeutisch akzeptables Salz davon nach Anspruch 1, wobei:
R₁ Phenyl ist,
wobei R₁ substituiert wird mit: C₁₋₄-Alkyl substituiert mit N(C₁₋₄-Alkyl)₂, C₁₋₄-Alkoxy substituiert mit N(C₁₋₄-Alkyl)₂, N(C₁₋₄-Alkyl)₂, Morpholino, Morpholinocarbonyl, Phenoxy, -SO₂-Piperidinyl, Piperazinyl substituiert mit C₁₋₄-Alkyl, Pyrazolyl substituiert mit zwei C₁₋₄-Alkylgruppen oder Tetrazolyl substituiert mit C₁₋₄-Alkyl.

5. Verbindung oder ein pharmazeutisch akzeptables Salz davon nach Anspruch 1, wobei:
R₁ Pyrazolyl ist,
wobei R₁ substituiert wird mit: C₁₋₄-Alkyl, C₁₋₄-Alkyl substituiert mit Morpholino, C₁₋₄-Halogenalkyl oder Benzyl substituiert mit C₁₋₄-Alkoxy.

6. Verbindung oder ein pharmazeutisch akzeptables Salz davon nach Anspruch 1, wobei:
R₁ unsubstituiert ist oder Benzothiazolyl, substituiert mit Halogen;
Imidazo[4,5-b]pyridinyl, substitutiert mit C₁₋₄-Alkyl;
Isoxazolo[5,4-b]pyridinyl, substituiert mit C₁₋₄-Alkyl;
unsubstituiertes Naphthyl;
Pyridinyl, substituiert mit Morpholino; oder
unsubstituiertes Chinolinyl ist.

7. Verbindung oder ein pharmazeutisch akzeptables Salz davon nach Anspruch 1, wobei:
R₂ eine beliebige Auswahl aus der Gruppe ist, bestehend aus Folgendem:

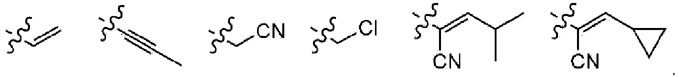

8. Verbindung oder ein pharmazeutisch akzeptables Salz davon nach Anspruch 1, wobei:
R₃ Wasserstoff, Fluor, Chlor, Methyl, Trifluormethyl oder Methoxy ist.

9. Verbindung oder ein pharmazeutisch akzeptables Salz davon nach Anspruch 1, wobei:
die Verbindung, die durch die chemische Formel 1 dargestellt wird, eine beliebige Verbindung ist, die aus der Gruppe ausgewählt wird, bestehend aus:
1) N-(3-(2-(4-(4-Methylpiperazin-1-yl)phenylamino)-[1,2,4]triazolo[1,5-a]pyridin-5-yloxy)phenyl)acrylamid,
2) N-(3-(2-(1-Methyl-1H-pyrazol-4-ylamino)-[1,2,4]triazolo[1,5-a]pyridin-5-yloxy)phenyl)acrylamid,
3) N-(3-(2-(Benzo[d]thiazol-6-ylamino)-[1,2,4]triazol[1,5-a]pyridin-5-yloxy)phenyl)acrylamid,
4) N-(3-(2-(4-Morpholinophenylamino)-[1,2,4]triazolo[1,5-a]pyridin-5-yloxy)phenyl)acrylamid,
5) N-(3-(2-(3-Phenoxyphenylamino)-[1,2,4]triazolo[1,5-a]pyridin-5-yloxy)phenyl)acrylamid,
6) N-(3-(2-(4-(Dimethylamino)phenylamino)-[1,2,4]triazolo[1,5-a]pyridin-5-yloxy)phenyl)acrylamid,
7) N-(3-(2-(3-(5-Methyl-1H-tetrazol-1-yl)phenylamino)-[1,2,4]triazolo[1,5-a]pyridin-5-yloxy)phenyl)acrylamid,
8) N-(3-(2-(1-(Difluormethyl)-1H-pyrazol-4-ylamino)-[1,2,4]triazolo[1,5-a]pyridin-5-yloxy)phenyl)acrylamid,
9) N-(3-(2-(3-(3,5-Dimethyl-1H-pyrazol-1-yl)phenylamino)-[1,2,4]triazolo[1,5-a]pyridin-5-yloxy)phenyl)acrylamid,
10) N-(3-(2-(4-(3,5-Dimethyl-1H-pyrazol-1-yl)phenylamino)-[1,2,4]triazolo[1,5-a]pyridin-5-yloxy)phenyl)acrylamid,
11) N-(3-(2-(2-Chlorbenzo[d]thiazol-6-ylamino)-[1,2,4]triazol[1,5-a]pyridin-5-yloxy)phenyl)acrylamid,
12) N-(3-(2-(Benzo[d]thiazol-5-ylamino)-[1,2,4]triazol[1,5-a]pyridin-5-yloxy)phenyl)acrylamid,
13) N-(3-(2-(4-Phenoxyphenylamino)-[1,2,4]triazolo[1,5-a]pyridin-5-yloxy)phenyl)acrylamid,
14) N-(3-(2-(1-(3-Methoxybenzyl)-1H-pyrazol-4-ylamino)-[1,2,4]triazolo[1,5-a]pyridin-5-yloxy)phenyl)acrylamid,
15) N-(3-(2-(4-(2-(Diethylamino)ethoxy)phenylamino)-[1,2,4]triazolo[1,5-a]pyridin-5-yloxy)phenyl)acrylamid,
16) N-(3-(2-(4-((Dimethylamino)methyl)phenylamino)-[1,2,4]triazolo[1,5-a]pyridin-5-yloxy)phenyl)acrylamid,
17) N-(3-(2-(4-(Morpholin-4-carbonyl)phenylamino)-[1,2,4]triazolo[1,5-a]pyridin-5-yloxy)phenyl)acrylamid,
18) N-(3-(2-(4-(Piperidin-1-ylsulfonyl)phenylamino)-[1,2,4]triazolo[1,5-a]pyridin-5-yloxy)phenyl)acrylamid,
19) N-(3-(2-(1-(2-Morpholinoethyl)-1H-pyrazol-4-ylamino)-[1,2,4]triazolo[1,5-a]pyridin-5-yloxy)phenyl)acrylamid,
20) N-(3-(2-(4-(4-Methylpiperazin-1-yl)phenylamino)-[1,2,4]triazolo[1,5-a]pyridin-5-yloxy)phenyl)but-2-ynamid,
21) N-(3-(2-(6-Morpholinopyridin-3-ylamino)-[1,2,4]triazolo[1,5-a]pyridin-5-yloxy)phenyl)acrylamid,
22) N-(3-(2-(6-Chlorbenzo[d]thiazol-2-ylamino)-[1,2,4]triazol[1,5-a]pyridin-5-yloxy)phenyl)acrylamid,
23) N-(3-(2-(Naphthalin-1-ylamino)-[1,2,4]triazol[1,5-a]pyridin-5-yloxy)phenyl)acrylamid,
24) N-(3-(2-(Chinolin-3-ylamino)-[1,2,4]triazolo[1,5-a]pyridin-5-yloxy)phenyl)acrylamid,
25) N-(3-(2-(3-Methylisoxazolo[5,4-b]pyridin-5-ylamino)-[1,2,4]triazolo[1,5-a]pyridin-5-yloxy)phenyl)acrylamid,
26) N-(3-(2-(1-Methyl-1H-imidazo[4,5-b]pyridin-6-ylamino)-[1,2,4]triazolo[1,5-a]pyridin-5-yloxy)phenyl)acrylamid,
27) N-(4-Fluor-3-(2-(4-morpholinophenylamino)-[1,2,4]triazol[1,5-a]pyridin-5-yloxy)phenyl)acrylamid,
28) N-(3-(2-(Benzo[d]thiazol-6-ylamino)-[1,2,4]triazolo[1,5-a]pyridin-5-yloxy)-4-fluorphenyl)acrylamid,
29) N-(4-Fluor-3-(2-(4-(4-methylpiperazin-1-yl)phenylamino)-[1,2,4]triazolo[1,5-a]pyridin-5-yloxy)phenyl)acrylamid,
30) N-(4-Fluor-3-(2-(1-methyl-1H-pyrazol-4-ylamino)-[1,2,4]triazolo[1,5-a]pyridin-5-yloxy)phenyl)acrylamid, und
31) N-(3-(2-(1-(Difluormethyl)-1H-pyrazol-4-ylamino)-[1,2,4]triazolo[1,5-a]pyridin-5-yloxy)-4-fluorphenyl)acrylamid.

10. Pharmazeutische Zusammensetzung zur Verwendung bei der Prävention oder Behandlung von entzündlichen Erkrankungen, Autoimmunerkrankungen, proliferativen Erkrankungen, hyperproliferativen Erkrankungen, immunvermittelten Erkrankungen, Krebs oder Tumoren, umfassend die Verbindung nach einem der Ansprüche 1 bis 9 oder ein pharmazeutisch akzeptables Salz davon.

## Revendications

1. Composé représenté par la Formule Chimique 1 suivante, ou un sel pharmaceutiquement acceptable de celui-ci : 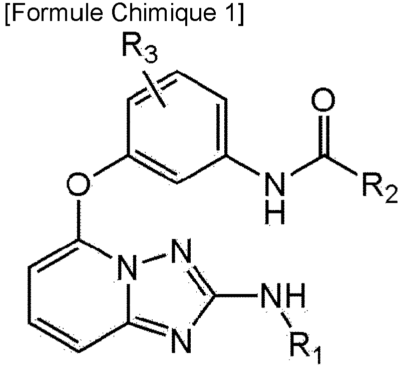 dans la Formule Chimique 1,
R₁ est un aryle en C_{6 à 10}, ou un hétéroaryle en C_{3 à 10} contenant 1 à 3 hétéroatomes chacun choisi indépendamment dans le groupe constitué par N, O et S,
où R₁ est non substitué, ou substitué par : un alkyle en C_{1 à 4}, un alkyle en C_{1 à 4} substitué par N(alkyle en C_{1 à 4})₂, un alkyle en C_{1 à 4} substitué par un morpholino, un haloalkyle en C_{1 à 4}, un halogène, un alkoxy en C_{1 à 4}, un alkoxy en C_{1 à 4} substitué par N(alkyle en C_{1 à 4})₂, un N(alkyle en C_{1 à 4})₂, un morpholino, un morpholinocarbonyle, un phénoxy, un pipéridinyle, un -SO₂-pipéridinyle, un pipérazinyle, un pipérazinyle substitué par un alkyle en C_{1 à 4}, un benzyle, un benzyle substitué par un alkoxy en C_{1 à 4}, un pyrazolyle, un pyrazolyle substitué par un ou deux alkyles en C_{1 à 4}, un tétrazolyle, ou un tétrazolyle substitué par un alkyle en C_{1 à 4},
R₂ est un alkyle en C_{1 à 5}, un alcényle en C_{2 à 5}, ou un alcynyle en C_{2 à 5}, où R₂ est non substitué, ou substitué par un ou deux substituants choisis dans le groupe constitué par un halogène, un cyano, un cycloalkyle en C_{3 à 6} et un alkyle en C_{1 à 5} substitué par un cyano, et
R₃ est un hydrogène, un halogène, un alkyle en C_{1 à 4}, un haloalkyle en C_{1 à 4}, ou un alcoxy en C_{1 à 4}.

2. Composé ou un sel pharmaceutiquement acceptable de celui-ci selon la revendication 1, dans lequel :
la Formule Chimique 1 est représentée par la Formule Chimique 1-1 suivante : 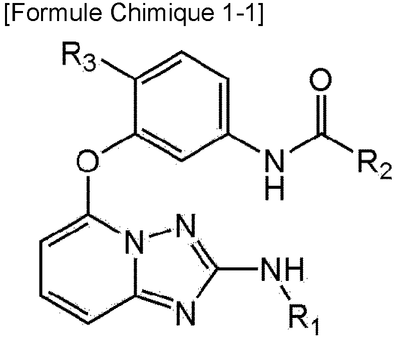 dans la Formule Chimique 1-1,
R₁ à R₃ sont tels que définis dans la revendication 1.

3. Composé ou un sel pharmaceutiquement acceptable de celui-ci selon la revendication 1, dans lequel :
R₁ est un benzothiazolyle, un imidazo[4,5-b]pyridinyle, un isoxazolo[5,4-b]pyridinyle, un naphtyle, un phényle, un pyrazolyle, un pyridinyle ou un quinolinyle.

4. Composé ou un sel pharmaceutiquement acceptable de celui-ci selon la revendication 1, dans lequel :
R₁ est un phényle,
dans lequel R₁ est substitué par : un alkyle en C_{1 à 4} substitué par N(alkyle en C_{1 à 4})₂, un alcoxy en C_{1 à 4} substitué par N(alkyle en C_{1 à 4})₂, un N(alkyle en C_{1 à 4})₂, un morpholino, un morpholinocarbonyle, un phénoxy, un -SO₂-pipéridinyle, un pipérazinyle substitué par un alkyle en C_{1 à 4}, un pyrazolyle substitué par deux alkyles en C_{1 à 4} ou un tétrazolyle substitué par un alkyle en C_{1 à 4}.

5. Composé ou un sel pharmaceutiquement acceptable de celui-ci selon la revendication 1, dans lequel :
R₁ est un pyrazolyle,
dans lequel R₁ est substitué par : un alkyle en C_{1 à 4}, un alkyle en C_{1 à 4} substitué par un morpholino, un haloalkyle en C_{1 à 4}, ou un benzyle substitué par un alcoxy en C_{1 à 4}.

6. Composé ou un sel pharmaceutiquement acceptable de celui-ci selon la revendication 1, dans lequel :
R₁ est non substitué, ou est un benzothiazolyle substitué par un halogène un imidazo[4,5-b]pyridinyle substitué par un alkyle en C_{1 à 4} ;
un isoxazolo[5,4-b]pyridinyle substitué par un alkyle en C_{1 à 4} ;
un naphtyle non substitué ;
un pyridinyle substitué par un morpholino ; ou
un quinolinyle non substitué.

7. Composé ou un sel pharmaceutiquement acceptable de celui-ci selon la revendication 1, dans lequel :
R₂ est l'un quelconque choisi dans le groupe consistant en ce qui suit :

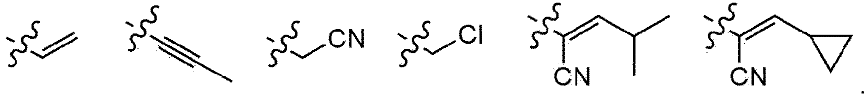

8. Composé ou un sel pharmaceutiquement acceptable de celui-ci selon la revendication 1, dans lequel :
R₃ est un hydrogène, un fluor, un chloro, un méthyle, un trifluorométhyle ou un méthoxy.

9. Composé ou un sel pharmaceutiquement acceptable de celui-ci selon la revendication 1, dans lequel :
le composé représenté par la Formule Chimique 1 est l'un quelconque choisi dans le groupe constitué par :
1) N-(3-(2-(4-(4-méthylpipérazin-1-yl)phénylamino)-[1,2,4]triazolo[1,5-a]pyridin-5-yloxy)phényl)acrylamide,
2) N-(3-(2-(1-méthyl-1H-pyrazol-4-ylamino)-[1,2,4]triazolo[1,5-a]pyridin-5-yloxy)phényl)acrylamide,
3) N-(3-(2-(benzo[d]thiazol-6-ylamino)-[1,2,4]triazolo[1,5-a]pyridin-5-yloxy)phényl)acrylamide,
4) N-(3-(2-(4-morpholinophénylamino)-[1,2,4]triazolo[1,5-a]pyridin-5-yloxy)phényl)acrylamide,
5) N-(3-(2-(3-phénoxyphénylamino)-[1,2,4]triazolo[1,5-a]pyridin-5-yloxy)phényl)acrylamide,
6) N-(3-(2-(4-(diméthylamino)phénylamino)-[1,2,4]triazolo[1,5-a]pyridin-5-yloxy)phényl)acrylamide,
7) N-(3-(2-(3-(5-méthyl-1H-tétrazol-1-yl)phénylamino)-[1,2,4]triazolo[1,5-a]pyridin-5-yloxy)phényl)acrylamide,
8) N-(3-(2-(1-(difluorométhyl)-1H-pyrazol-4-ylamino)-[1,2,4]triazolo[1,5-a]pyridin-5-yloxy)phényl)acrylamide,
9) N-(3-(2-(3-(3,5-diméthyl-1H-pyrazol-1-yl)phénylamino)-[1,2,4]triazolo[1,5-a]pyridin-5-yloxy)phényl)acrylamide,
10) N-(3-(2-(4-(3,5-diméthyl-1H-pyrazol-1-yl)phénylamino)-[1,2,4]triazolo[1,5-a]pyridin-5-yloxy)phényl)acrylamide,
11) N-(3-(2-(2-chlorobenzo[d]thiazol-6-ylamino)-[1,2,4]triazolo[1,5-a]pyridin-5-yloxy)phényl)acrylamide,
12) N-(3-(2-(benzo[d]thiazol-5-ylamino)-[1,2,4]triazolo[1,5-a]pyridin-5-yloxy)phényl)acrylamide,
13) N-(3-(2-(4-phénoxyphénylamino)-[1,2,4]triazolo[1,5-a]pyridin-5-yloxy)phényl)acrylamide,
14) N-(3-(2-(1-(3-méthoxybenzyl)-1H-pyrazol-4-ylamino)-[1,2,4]triazolo[1,5-a]pyridin-5-yloxy)phényl)acrylamide,
15) N-(3-(2-(4-(2-(diéthylamino)éthoxy)phénylamino)-[1,2,4]triazolo[1,5-a]pyridin-5-yloxy)phényl)acrylamide,
16) N-(3-(2-(4-((diméthylamino)méthyl)phénylamino)-[1,2,4]triazolo[1,5-a]pyridin-5-yloxy)phényl)acrylamide,
17) N-(3-(2-(4-(morpholine-4-carbonyl)phénylamino)-[1,2,4]triazolo[1,5-a]pyridin-5-yloxy)phényl)acrylamide,
18) N-(3-(2-(4-(pipéridin-1-ylsulfonyl)phénylamino)-[1,2,4]triazolo[1,5-a]pyridin-5-yloxy)phényl)acrylamide,
19) N-(3-(2-(1-(2-morpholinoéthyl)-1H-pyrazol-4-ylamino)-[1,2,4]triazolo[1,5-a]pyridin-5-yloxy)phényl)acrylamide,
20) N-(3-(2-(4-(4-méthylpipérazin-1-yl)phénylamino)-[1,2,4]triazolo[1,5-a]pyridin-5-yloxy]phényl)but-2-ynamide,
21) N-(3-(2-(6-morpholinopyridin-3-ylamino)-[1,2,4]triazolo[1,5-a]pyridin-5-yloxy)phényl)acrylamide,
22) N-(3-(2-(6-chlorobenzo[d]thiazol-2-ylamino)-[1,2,4]triazolo[1,5-a]pyridin-5-yloxy)phényl)acrylamide,
23) N-(3-(2-(naphtalén-1-ylamino)-[1,2,4]triazolo[1,5-a]pyridin-5-yloxy)phényl)acrylamide,
24) N-(3-(2-(quinolin-3-ylamino)-[1,2,4]triazolo[1,5-a]pyridin-5-yloxy)phényl)acrylamide,
25) N-(3-(2-(3-méthylisoxazolo[5,4-b]pyridin-5-ylamino)-[1,2,4]triazolo[1,5-a]pyridin-5-yloxy)phényl)acrylamide,
26) N-(3-(2-(1-méthyl-1H-imidazo[4,5-b]pyridin-6-ylamino)-[1,2,4]triazolo[1,5-a]pyridin-5-yloxy)phényl)acrylamide,
27) N-(4-fluoro-3-(2-(4-morpholinophénylamino)-[1,2,4]triazolo[1,5-a]pyridin-5-yloxy)phényl)acrylamide,
28) N-(3-(2-(benzo[d]thiazol-6-ylamino)-[1,2,4]triazolo[1,5-a]pyridin-5-yloxy)-4-fluorophényl)acrylamide,
29) N-(4-fluoro-3-(2-(4-(4-méthylpipérazin-1-yl)phénylamino)-[1,2,4]triazolo[1,5-a]pyridin-5-yloxy)phényl)acrylamide,
30) N-(4-fluoro-3-(2-(1-méthyl-1H-pyrazol-4-ylamino)-[1,2,4]triazolo[1,5-a]pyridin-5-yloxy)phényl)acrylamide, et
31) N-(3-(2-(1-(difluorométhyl)-1H-pyrazol-4-ylamino)-[1,2,4]triazolo[1,5-a]pyridin-5-yloxy)-4-fluorophényl)acrylamide.

10. Composition pharmaceutique pour utilisation dans la prévention ou le traitement d'une maladie inflammatoire, d'une maladie auto-immune, d'une maladie proliférative, d'une maladie hyperproliférative, d'une maladie à médiation immunitaire, d'un cancer ou d'une tumeur, comprenant le composé selon l'une quelconque des revendications 1 à 9 ou un sel pharmaceutiquement acceptable de celui-ci.
